# EUROPEAN PATENT APPLICATION

(11) **EP 3 441 465 A1**
(43) Date of publication of application: **13.02.2019**
(21) Application number: 17779251.2
(22) Date of filing: 04.04.2017
(51) Int. Cl.: C12N 15/09, A61K 39/395, A61K 45/00, A61K 47/50, A61P 35/00, C07K 16/30, C12N 5/16

(54) **CANCER TREATMENT PHARMACEUTICAL COMPOSITION USING ANTI-MCT5 ANTIBODY**

(30) Priority: 06.04.2016 JP 2016076565
(71) Applicant: Order-Made Medical Research Inc., Kashiwa-shi, Chiba 277-0882 (JP)
(72) Inventor: SATOFUKA, Hiroyuki, Kashiwa-shi Chiba 277-0882 (JP); OKABE, Youko, Kashiwa-shi Chiba 277-0882 (JP); KATO, Dai, Kashiwa-shi Chiba 277-0882 (JP)
(74) Representative: J A Kemp
(86) International application number: PCT/JP2017/014600
(87) International publication number: WO 2017/175874

(57) **Abstract**

The present invention aims to provide a cancer-specific new pharmaceutical composition with fewer side effects.

The present invention provides a pharmaceutical composition comprising a monoclonal antibody recognizing a polypeptide of an extracellular region of native MCT5.

## Description

### TECHNICAL FIELD

The present invention relates to a pharmaceutical composition for use in treatment of cancers including breast cancer and colorectal cancer, which comprises an antibody conjugate configured such that a monoclonal antibody recognizing an extracellular domain of MCT5 is conjugated with a compound having activity against cancers and other hyperproliferative diseases. The present invention also relates to the therapeutic use of the antibody in combination with such a compound or the therapeutic use of the antibody conjugate in combination with such a compound.

### BACKGROUND ART

Cancer ranks high in the causes of death all over the world. In particular, breast cancer is a woman's cancer most commonly seen in the United Kingdom, the United States and Japan. The number of women suffering from breast cancer in Japan has now exceeded 40,000 and still tends to increase. Recent advances in medical equipment for mammography or other diagnostic imaging techniques have allowed early diagnosis of breast cancer, and it is expected that the healing rate of breast cancer will gradually increase.

Patients who develop recurrence or metastasis are not few in number although they have undergone surgical excision and/or radiation therapy at an early stage of breast cancer as a result of early diagnosis. In fact, there is a report showing that about 40% of patients suffering from breast cancer will die although the five-year survival rate after primary breast cancer resection is 90% or more (Non-patent Document 1: Weigelt B, et al., Nature Reviews, 2005, 5, 591-602). Moreover, it is also reported that patients who have survived more than 20 years and are therefore diagnosed as having gone into remission account for only 2% to 3% of all patients. Namely, for treatment of breast cancer, there is a demand for the development of a new pharmaceutical preparation which allows suppression of recurrence or metastasis of breast cancer.

Some cases of breast cancer do not develop recurrence or metastasis, but these cases are difficult to predict in advance; and hence many patients are now administered with therapeutic agents for the purpose of reducing the risk of recurrence or metastasis. For patients who do not develop recurrence or metastasis, this means that they receive unwanted treatment which may cause side effects.

On the other hand, in breast cancer surgery, conservative therapy is conducted for the purpose of improving the quality of life (QOL) after surgery. As of 2001, conservative therapy has been conducted in 40% or more of all patients. However, among patients who have undergone conservative therapy, some patients are at high risk of recurrence or metastasis.

The "recurrence" of cancer refers to an event where after surgical excision of the primary focus, invisible small cancer lesions are left behind without being removed and then will form tumors again, or an event where cancer lesions whose size has been reduced upon drug therapy (treatment with anticancer agents) and/or radiation therapy will become larger again. Such an event when occurring in close proximity to the first occurring cancer is referred to as "recurrence." On the other hand, "metastasis" refers to an event where cancer cells reach an organ different from the primary focus and cause secondary formation of cancer of the same type. For example, a case where breast cancer forms tumors again in mammae is "recurrence," while secondary cancer having metastasized to lungs is breast cancer lung "metastasis" formed by malignant breast cancer cells.

As to the mechanism by which "recurrence" or "metastasis" occurs, the major cause is probably that cancer cells having high invasive capacity invade another site in the same tissue as the primary focus or locally invade cancer adjacent tissues and are left behind without being removed by surgical operation, etc.

When cancer cells cause recurrence or metastasis even after surgical removable of cancer sites, the character of these cancer cells is critical. As indicators representing the poor character of cancer cells and the degree of progression, the grade of atypism and the depth of invasion are used. The grade of atypism indicates what degree of morphological difference exists between cancer cells and normal cells. On the other hand, the depth of invasion indicates the depth of cancer. When cancer cells have high invasive capacity, the depth of invasion is larger and these cancer cells are classified as more malignant cancer cells. For this reason, the depth of invasion is used for clinical stage classification of malignant tumors. Accordingly, cancer cells having high invasive capacity are highly malignant and considered to be cancer at high risk of recurrence or metastasis. Namely, cancer cells having high invasive capacity cause an increase in the risk of recurrence or metastasis. Accordingly, when treatment is conducted to target cancer cells having high invasive capacity, cases of recurrence or metastasis can be reduced in number, thus allowing an expectation that the mortality can be reduced.

For treatment of various cancers including breast cancer and colorectal cancer, surgical treatment and chemotherapy are commonly used, while attempts have also been made to search for cancer-specific new therapies since the recent appearance of molecular targeted drugs.

As an antibody drug approved in Japan for use as a molecular targeted drug for breast cancer, Herceptin is known. Herceptin is an antibody drug targeted at a receptor called HER2 (also referred to as ERBB2 or Her2/neu), which has been approved for use as a therapeutic agent for metastatic breast cancer with HER2 overexpression and is considered to kill cancer cells via a mechanism of action which involves binding to HER2 on the cell membrane to cause antibody-dependent cellular cytotoxicity (ADCC) mediated by natural killer cells (NK cells) and/or macrophages, etc., whereby the cancer cells will be killed.

As antibody drugs for colorectal cancer, Avastin and Erbitux are known. They are antibody drugs targeted at growth factors such as VEGF and EGF. Avastin has been approved for use in progressive and recurrent colorectal cancer for which curative resection is impossible. Avastin exerts an anticancer effect via a mechanism of action which involves binding to VEGF to prevent its binding to VEGF receptors, thereby inhibiting vascularization and blocking nutrition to tumor tissues. Erbitux is intended to stop the proliferation of cancer cells through binding to EGF receptors and thereby inhibiting EGF-mediated cell proliferation signals.

As described above, molecular targeted drugs, typified by antibodies, are superior agents in terms of killing cancer cells upon specifically recognizing cancer. However, molecular targeted drugs will cause serious side effects in some cases when antibodies also bind to normal cells. For example, Herceptin, which is a therapeutic agent for breast cancer, may cause not only headache, asthenia, nausea and vomiting, but also interstitial pneumonia, bone marrow inhibition, hepatic disorders, renal disorders and cerebrovascular disorders. Moreover, in tissue staining, Herceptin is also known to strongly react with normal cardiomyocytes to thereby cause severe cardiac disorders (Non-patent Document 2: British Journal of Cancer, 94, 1016-1020, 2006). Further, Herceptin is an antibody drug targeted at Her2, and hence there remains a problem in that Herceptin is effective only for patients who express Her2.

In the case of Avastin, which is a therapeutic agent for colorectal cancer, its side effects include hemorrhage, thrombosis, gastrointestinal perforation, delayed wound healing, increased blood pressure and so on, among which thrombosis and gastrointestinal perforation are fatal side effects (Non-patent Document 3: Cancer Research, 57, 4593-4599, 1997). Side effects known for Erbitux include skin disorders and so on, which are not fatal but cause itching and white pustules, resulting in mental and physical burdens on patients (Non-patent Document 4: Journal of Clinical Oncology, 22, 1201-1208, 2004). Moreover, Erbitux also has a problem in that it has no effect on canceration caused by a change in signals downstream of EGF receptors (e.g., K-ras mutation).

In view of the foregoing, antibody drugs against cancers still have problems, e.g., in that they will cause severe side effects and are effective only for limited patients. Thus, there has been a demand for new development of cancer-specific molecular targets and pharmaceutical preparations with fewer side effects.

When compared to normal cells, cancer cells are characterized not only by having high proliferative ability, but also by having no limit on the number of cell divisions and causing invasion and/or metastasis to surrounding tissues. Recent studies have indicated that some limited number, but not all, of the cancer cells in cancer tissue have such properties. Namely, these limited number of cancer cells have self-replication ability (i.e., the ability to produce completely the same cells as themselves) and pluripotency (i.e., the ability to differentiate into many different cell types), which are characteristics common to stem cells including embryonic stem cells and somatic stem cells. Due to these characteristics, cancer cells would act not only to maintain the same cells as themselves in cancer tissue through self-replication, but also to generate the great majority of surrounding cancer cells as a result of differentiation. Such a limited number of cancer cells are called cancer stem cells, and there has been proposed a hypothesis that cancer occurs and progresses from these stem cell-like cells (cancer stem cell hypothesis).

Currently known cancer stem cell markers include molecular markers such as CD133, CD24, CD44 and so on (Non-patent Document 5: GASTROENTEROLOGY, 138, 2151-2162, 2010). However, antibodies against these markers bind to only some cancer stem cells and are therefore regarded as having no efficacy as therapeutic agents. On the other hand, a marker called LGR5 is also known. This marker has a mechanism to activate Wnt/β-catenin signals through interaction with R-spondin and is therefore suggested to have the potential to be used as a cancer stem cell marker (Patent Document 1: JP 2010-532169 A).

Cancer stem cells are considered to be a major factor for cancer recurrence and/or metastasis, and the importance of targeting cancer stem cells in cancer treatment has been pointed out. However, cancer stem cells constitute only a few percentages in tumor tissue, and hence therapeutic agents designed to target only cancer stem cells would not be able to kill cancer cells in general. Namely, for cancer medication, it is an important problem to develop a new therapy targeting a marker which is expressed at an extremely higher level in cancer stem cells than in normal tissues and is also expressed in ordinary cancer cells.

There is a mechanism by which a monoclonal antibody against a molecule specifically expressed in particular cancer cells is taken up into the cells after binding to a membrane protein serving as an antigen on the cell surface. This event is referred to as internalization. Internalization is originally a kind of cellular signaling mechanism which occurs upon ligand-receptor binding, and is reported to occur not only in hormone and cytokine receptors, but also in transporters. By taking advantage of the finding that antibody binding causes internalization, efforts have been made to pursue the development of a new pharmaceutical mixture of a molecular targeted drug and another drug, which is designed as an antibody conjugated with a drug to thereby target cells specifically expressing a target molecule. Such a pharmaceutical mixture is referred to as an antibody-drug conjugate (ADC).

Among ADCs, a conjugate formed between Herceptin (trastuzumab) and DM1 (taxane-based drug), i.e., kadcyla (trastuzumab emtansine or T-DM1) has been approved as a therapeutic agent for HER2-positive advanced breast cancer. As to the therapeutic mechanism of this conjugate, there is a report showing that DM1 serving as an anticancer agent can effectively kill HER2-positive cells when taken up into the cells (Non-patent Document 6: Cancer Res 2008; 68: (22). November 15, 2008).

Membrane proteins include not only receptors binding to their ligands, but also transport proteins (hereinafter referred to as transporters) which allow active or passive transport of low molecular compounds such as amino acids and sugars. These molecules have been considered not to cause internalization because they are responsible for allowing permeation of molecules inside and outside of cells. However, as shown in Non-patent Document 7 (The Journal of Biological Chemistry, 285, 27289-27301, 2010), SLC6A3 (Sodium depended dopamine transporter) is reported to cause internalization constitutively; and hence not only receptors, but also transporters have the potential to allow efficient transport of antibody-conjugated drugs into cells.

MCT5 (Monocarboxylate transporter 5) is a membrane protein composed of 487 amino acids and registered under NCBI (National Center for Biotechnology Information) Reference Sequences [RefSeq] ID: NM_004696.2 and NP_004687.1 (SEQ ID NO: 1: nucleotide sequence, SEQ ID NO: 2: amino acid sequence). MCT5 is an orphan transporter whose target substance to be transported has not been identified, and is classified as a member of the MCT group based on the homology of its amino acid sequence.

### Patent Documents

Patent Document 1: JP 2010-532169 A

### Non-patent Documents

Non-patent Document 1: Nature Reviews, 5, 591-602, 2005
Non-patent Document 2: British Journal of Cancer, 94, 1016-1020, 2006
Non-patent Document 3: Cancer Research, 57, 4593-4599, 1997
Non-patent Document 4: Journal of Clinical Oncology, 22, 1201-1208, 2004
Non-patent Document 5: GASTROENTEROLOGY, 138, 2151-2162, 2010
Non-patent Document 6: Cancer Research, 68, 9280-90, 2008
Non-patent Document 7: The Journal of Biological Chemistry, 285, 27289-27301, 2010

### DISCLOSURE OF THE INVENTION

In general, surgical treatment of cancer has problems not only in that it is difficult to treat metastatic lesions, but also in that it involves invasion and occurrence of complications. Moreover, chemotherapy and radiation therapy have problems of side effects. Further, existing antibody drugs not only cause side effects, but also have no effect on some cancers. For these reasons, there has been a demand for the development of cancer-specific molecular targets and new pharmaceutical preparations with fewer side effects.

As a result of repeating extensive and intensive efforts to solve the problems stated above, the inventors of the present invention have found that when cells are reacted with an anti-MCT5 monoclonal antibody recognizing an extracellular domain of MCT5 expressed at a higher level in highly invasive cancers than in ordinary cancer cells, this antibody is internalized into the cells at least through the clathrin pathway. Namely, the inventors of the present invention have found that a conjugate formed between such an anti-MCT5 monoclonal antibody and an anticancer agent allows effective killing of cancer cells. This finding led to the completion of the present invention.

Namely, the present invention is as follows.
(1) A monoclonal antibody against MCT5 or a fragment thereof.
(2) The antibody according to (1) above, wherein the fragment of MCT5 is a fragment of an extracellular region of MCT5.
(3) The antibody according to (1) or (2) above, wherein the fragment of the extracellular region of MCT5 consists of the amino acid sequence shown in SEQ ID NO: 4.
(4) The antibody according to any one of (1) to (3) above, wherein the antibody is a chimeric antibody, a reshaped human antibody or a humanized antibody.
(5) An antibody binding to an antigenic determinant to which the antibody according to any one of (1) to (4) above binds.
(6) A fragment of the antibody according to any one of (1) to (5) above.
(7) A hybridoma producing the antibody according to any one of (1) to (4) above.
(8) A pharmaceutical composition for use in cancer treatment, which comprises the antibody according to any one of (1) to (5) above or the fragment according to (6) above.
(9) A pharmaceutical composition for use in cancer treatment, which comprises the antibody according to any one of (1) to (5) above or the fragment according to (6) above in combination with a chemotherapeutic agent, a toxin or a radioisotope.
(10) A pharmaceutical composition for use in cancer treatment, which comprises a conjugate of the antibody according to any one of (1) to (5) above or the fragment according to (6) above with a chemotherapeutic agent, a toxin or a radioisotope.

The present invention provides an anti-MCT5 monoclonal antibody or an antigen-binding fragment thereof, and a pharmaceutical composition comprising a substance composed of such an antibody or antigen-binding fragment conjugated with an anticancer agent. Such a pharmaceutical composition is particularly useful as a pharmaceutical composition for use in treatment of breast cancer and colorectal cancer.

Moreover, MCT5 is expressed at a higher level in cancer cells having high invasive capacity than in normal tissues. Thus, the present invention provides a new agent or method for cancer treatment, which is capable of targeting highly malignant cancer cells to thereby suppress cancer progression, metastasis and recurrence.

### BRIEF DESCRIPTION OF THE DRAWINGS

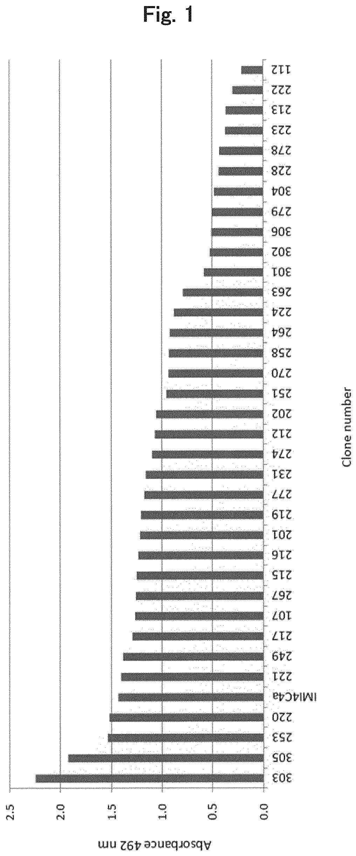

Figure 1 shows the results obtained when monoclonal antibodies produced by hybridoma cells were analyzed for their ability to react with ICD4, which is an antigen used for immunization of mice.

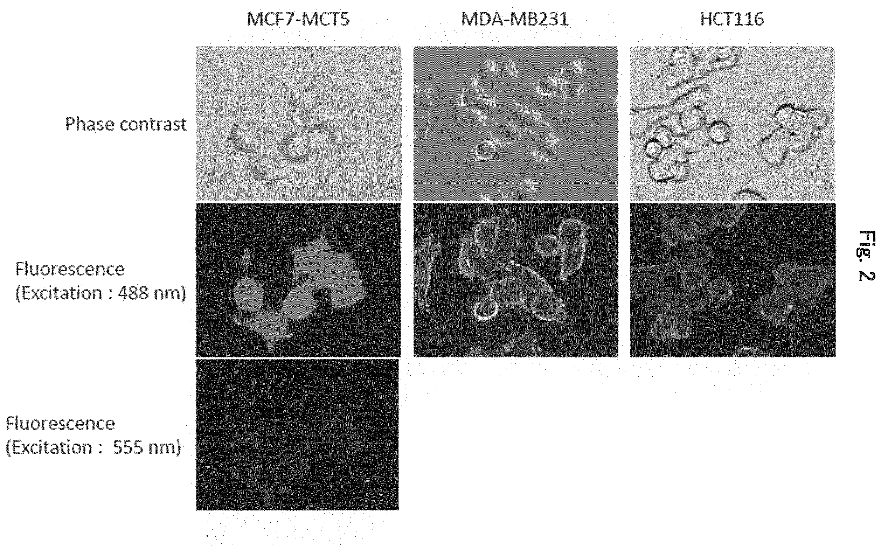

Figure 2 shows the results obtained when human breast cancer MCF7 cells engineered to overexpress MCT5 (MCF7-MCT5 cells), highly metastatic human breast cancer MDA-MB231 cells and human colorectal cancer HCT116 cells were analyzed for their reaction with anti-MCT5 antibody by immunocytological staining. During analysis, an Alexa 555-labeled secondary antibody was used as a secondary antibody for the MCF7-MCT5 cells, while an Alexa 488-labeled secondary antibody was used for the MDA-MB231 and HCT116 cells. Since the MCF7-MCT5 cells had been engineered to carry a gene designed such that EGFP is expressed simultaneously with MCT5, EGFP fluorescence was observed upon excitation at 488 nm.

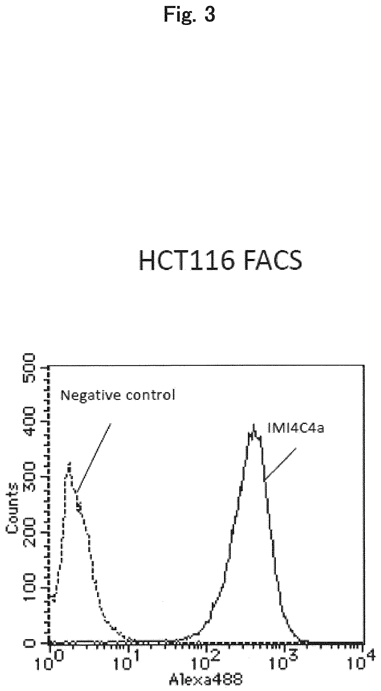

Figure 3 shows the results obtained when an anti-MCT5 monoclonal antibody of clone No. IMI4C4a was analyzed for its binding to the cell surface of human colorectal cancer HCT116 cells by using Flow cytometry, i.e., the results obtained when the cells and the antibody were reacted with each other and then reacted with an Alexa 488-labeled secondary antibody for analysis.

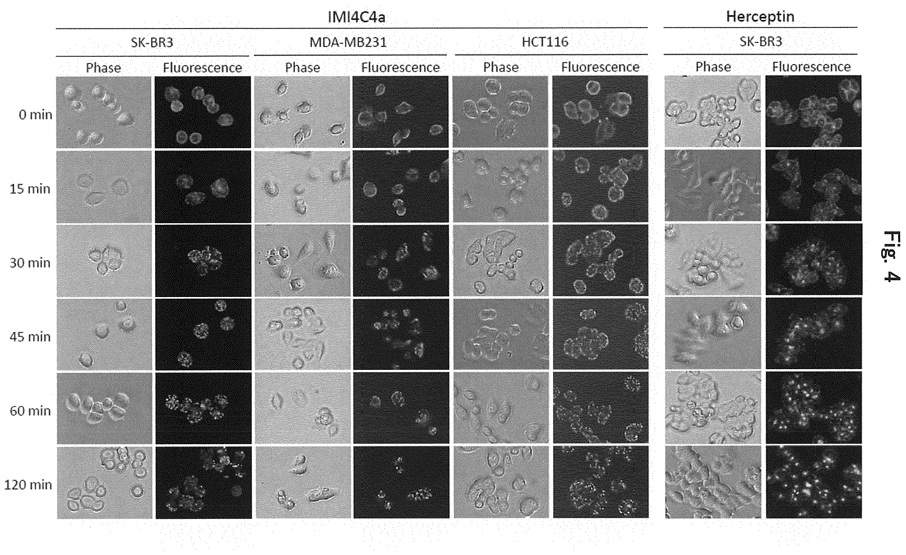

Figure 4 shows the results obtained when human breast cancer SK-BR3 cells and MDA-MB231 cells as well as human colorectal cancer HCT116 cells were reacted with the antibody IMI4C4a and reacted with an Alexa 488-labeled secondary antibody, and then analyzed over time for their uptake of the antibody. The time shown on the left side in the figure represents the culture period after reaction with the antibody. In addition, the right panel in the figure shows the results obtained when Herceptin and SK-BR3 cells were analyzed in the same manner. It is shown that the antibody (green fluorescence) is taken up into the cells in a time-dependent manner. Lysosomes (red fluorescence) are stained with a pH-sensitive fluorescent dye, LysoTracker DND-99.

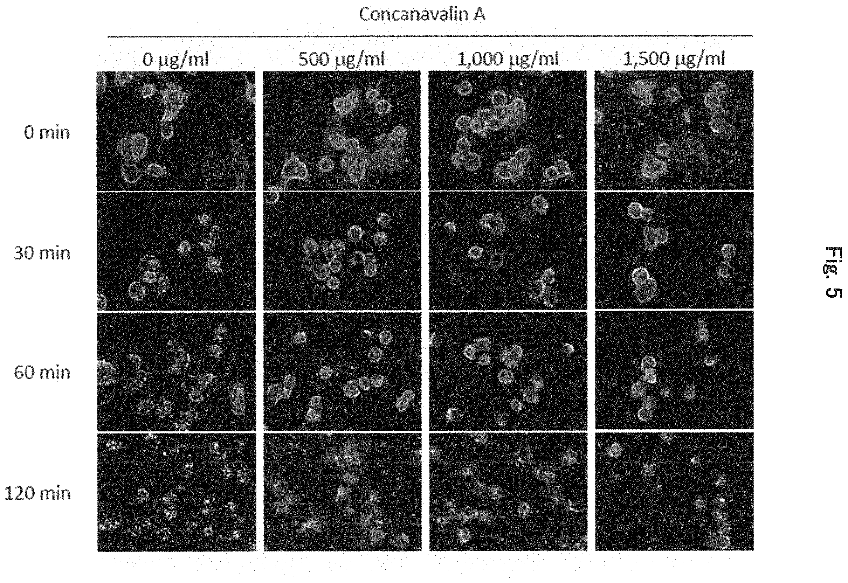

Figure 5 shows the results obtained when HCT116 cells were treated for 1 hour with Concanavalin A at the respective concentrations indicated at the top of the figure and then reacted with the antibody IMI4C4a to analyze time-dependent uptake into the cells.

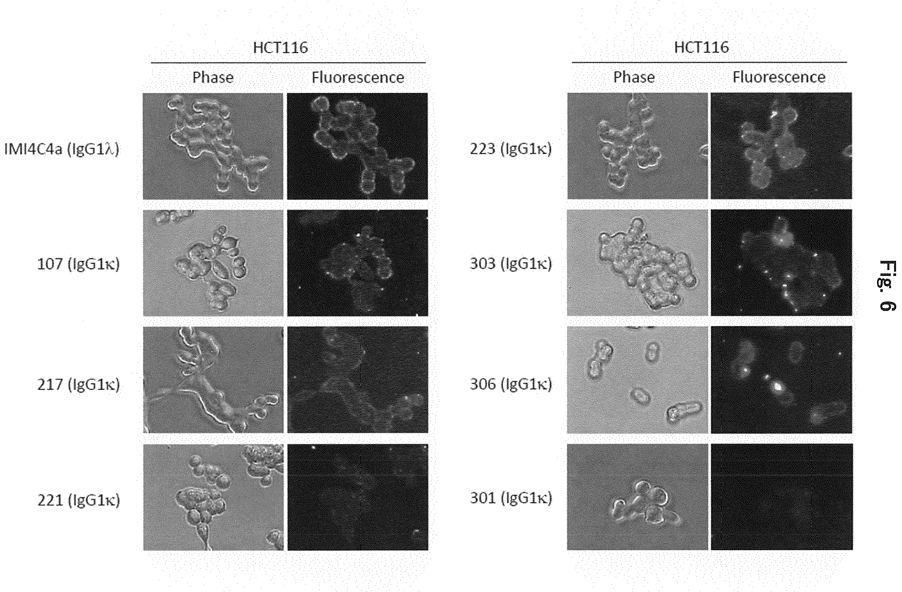

Figure 6 shows the results obtained when HCT116 cells were treated for 1 hour with Concanavalin A at 0.5 mg/ml and then reacted with antibodies IMI4C4a, 107, 217, 221, 223, 303, 306 and 301 to analyze the antibodies for their reactivity with the cells while being inhibited from uptake into the cells.

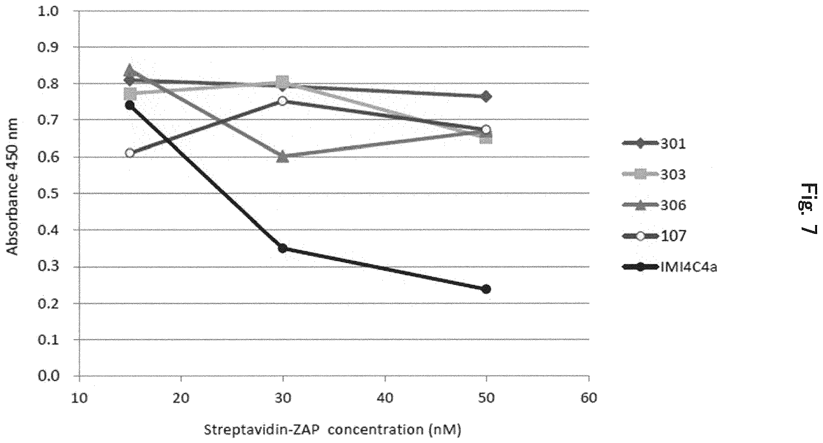

Figure 7 shows the analysis results of whether MCF7-MCT5 cells are inhibited from proliferation by antibody-saporin conjugates. The concentration indicated at the bottom of the figure represents the concentration of Streptavidin-ZAP (a Streptavidin-saporin conjugate) added to the medium. In addition, the biotinylated antibodies used were all adjusted to 50 nM.

### DESCRIPTION OF EMBODIMENTS

The present invention will be described in more detail below. It should be noted that the present invention is not limited to the following embodiments and can be implemented with modifications, as appropriate, within the spirit of the present invention.

The present invention relates to a monoclonal antibody binding to MCT5 or an antigen-binding fragment thereof. Moreover, the present invention relates to such an antibody or an antigen-binding fragment thereof, and a pharmaceutical composition comprising the same, as well as hybridoma cells producing the antibody, nucleic acids, recombinant expression vectors and cells for use in the preparation of the antibody and fragment.

The inventors of the present invention have found in their previous studies that MCT5 is expressed in breast cancer and colorectal cancer, and that a monoclonal antibody recognizing MCT5 can be used as a detection agent or diagnostic agent for colorectal cancer. Further, the inventors of the present invention have demonstrated that a region at positions 751 to 774 (AATTTAACAGTCTCACAAAATCAA (SEQ ID NO: 3)) in the nucleotide sequence of MCT5, i.e., a region at amino acid residues 251 to 258 (NLTVSQNQ (SEQ ID NO: 4)), which has been regarded as an intracellular domain, is located in the extracellular portion. In addition, based on the findings that human breast cancer cells engineered to overexpress MCT5 have increased invasive capacity, that an anti-MCT5 antibody (clone No. IMI4C4a) binds to only a portion of tumor tissue, and that anti-MCT5 antibody-positive tissue increases in proportion with advance in the stage of breast cancer tissue, the inventors of the present invention have demonstrated that MCT5 is specifically expressed in highly invasive breast cancer, and hence an anti-MCT5 antibody allows detection of highly malignant breast cancer. Furthermore, the inventors of the present invention have demonstrated that the antibody IMI4C4a has an epitope at positions 251 to 258 (SEQ ID NO: 4: amino acid sequence) in the MCT5 protein.

The present invention relates to a pharmaceutical composition comprising an antibody-anticancer agent conjugate configured such that a monoclonal antibody binding to MCT5 or an antigen-binding fragment thereof is conjugated with an anticancer agent.

MCT5 (Monocarboxylate transporter 5) is a multi-transmembrane protein and is an orphan transporter whose target substance to be transported has not been identified. In light of its amino acid sequence homology with MCT1 and MCT4 which are involved in the regulation of the lactate concentration between inside and outside of cells, MCT5 is predicted to have similar functions.

The present invention is based on the finding that the membrane protein MCT5 is a protein overexpressed in cancer tissues and is expressed at a higher level in highly invasive cancer cells serving as a major factor for cancer recurrence and/or metastasis than in ordinary cancer cells.

The present invention provides a monoclonal antibody recognizing an extracellular region of MCT5 or an antigen-binding fragment thereof. Since MCT5 is expressed in cancer cells such as breast cancer and colorectal cancer, this antibody specifically binds to cancer cells such as breast cancer and colorectal cancer.

### 1. Anti-MCT5 antibody of the present invention

The monoclonal antibody of the present invention (hereinafter also referred to as "the anti-MCT5 antibody of the present invention") is capable of recognizing native MCT5. The term "native" is intended to mean being in a state of retaining the intact three-dimensional structure which is taken by the intended protein in an *in vivo* environment.

In another embodiment of the present invention, the anti-MCT5 antibody of the present invention is capable of recognizing an extracellular region of MCT5. More specifically, the anti-MCT5 antibody of the present invention recognizes at least a part of the three-dimensional structure in the extracellular region of MCT5 as an epitope. In particular, the extracellular region is encoded by the nucleotide sequence of a region at positions 751 to 774 (SEQ ID NO: 3) in the nucleotide sequence of the MCT5 gene, and the anti-MCT5 antibody of the present invention is capable of recognizing a region at amino acid residues 251 to 258 (SEQ ID NO: 4).

Within the range of retaining the binding activity to a polypeptide having the amino acid sequence shown in SEQ ID NO: 4, i.e., as long as a target polypeptide has functions as an extracellular region of MCT5, the anti-MCT5 antibody of the present invention may also recognize a mutated polypeptide comprising substitution, deletion or insertion of one or several (e.g., 2, 3, 4 or 5) amino acids in the amino acid sequence shown in SEQ ID NO: 4 or a mutated polypeptide sharing an identity of 50% or more, preferably 62.5% or more, 75% or more, or 87.5% or more with the amino acid sequence shown in SEQ ID NO: 4.

In yet another embodiment of the present invention, the anti-MCT5 antibody of the present invention may recognize a polypeptide having functions as an extracellular region of MCT5 and being a polypeptide encoded by a polynucleotide consisting of the nucleotide sequence shown in SEQ ID NO: 3, a polypeptide encoded by a polynucleotide comprising the nucleotide sequence shown in SEQ ID NO: 3, a mutated polypeptide encoded by a polynucleotide sharing an identity of 70% or more, preferably 75% or more, 79% or more, 83% or more, 87.5% or more, 91% or more, or 95% or more with the nucleotide sequence shown in SEQ ID NO: 3, or a mutated polypeptide encoded by a polynucleotide comprising a nucleotide sequence hybridizable under high stringent conditions with the nucleotide sequence shown in SEQ ID NO: 3. For this purpose, hybridization may be accomplished in a known manner (e.g., Molecular Cloning 2nd Ed (Cold Spring Harbor Lab. Press, 1989). High stringent conditions refer to so-called conditions under which a specific hybrid is formed and any nonspecific hybrid is not formed. For example, high stringent conditions refer to conditions at a sodium concentration of 10 mM to 300 mM, preferably 20 mM to 100 mM and at a temperature of 25°C to 70°C, preferably 42°C to 55°C. Alternatively, since the anti-MCT5 antibody of the present invention binds to MCT5, among the above mutated polypeptides, those to which the anti-MCT5 antibody of the present invention is capable of binding indicate that the antibody retains its binding activity to polypeptides having the amino acid sequence shown in SEQ ID NO: 4, i.e., they fall within polypeptides having functions as an extracellular region of MCT5.

To confirm whether or not a mutated polypeptide has functions as an extracellular domain of MCT5, animal cells or other cells may be forced to express the mutated polypeptide and analyzed by invasion assay (e.g., a Matrigel invasion chamber) to determine whether or not the invasive capacity is increased. Moreover, such an extracellular region of MCT5 is a cell surface site of a marker protein whose expression is increased in cancer cells having high invasive capacity. To confirm whether or not a mutated polypeptide has functions as an extracellular domain of MCT5, the expression of the mutated polypeptide may be compared between normal cells and cancer cells by immunostaining, ELISA, immunoprecipitation, western blotting, Flow cytometry, etc.

Binding between the anti-MCT5 antibody of the present invention and its epitope or a mutated polypeptide thereof may be confirmed by ELISA, immunoprecipitation, western blotting, etc.

In yet another embodiment of the present invention, the anti-MCT5 antibody of the present invention also recognizes proteins encoded by mRNA variants of MCT5. The anti-MCT5 antibody of the present invention has the ability to bind not only to the full-length MCT5, but also to partially deficient mutants thereof, and is therefore capable of binding to a wide range of MCT5-expressing cancer cells.

In the context of the present invention, the term "antibody" refers to an immunoglobulin molecule composed of two heavy chains and two light chains. Each heavy chain is composed of a heavy chain variable region (VH) and a heavy chain constant region (CH). This heavy chain constant region consists of three domains CH1, CH2 and CH3. Each light chain is composed of a light chain variable region (VL) and a light chain constant region (CL). The light chain constant region consists of one domain CL. The heavy chain variable region and the light chain variable region are each composed of relatively conserved regions referred to as framework regions (FRs) and hyper variable regions referred to as complementarity determining regions (CDRs). VH and VL each consist of three CDRs and four FRs, which are arranged in the order of FR1, CDR1, FR2, CDR2, FR3, CDR3 and FR4 from the amino-terminal end to the carboxy-terminal end. The anti-MCT5 antibody of the present invention may be a full-length antibody or an antigen-binding fragment thereof.

In the context of the present invention, an "antigen-binding portion" of an antibody (or simply an "antigen portion") refers to one or more fragments of the antibody, each having the ability to specifically bind to its antigen (e.g., MCT5). It is known that the antigen-binding function of an antibody may be achieved by a fragment of the full-length antibody. Examples of such an "antigen-binding portion" of an antibody include, but are not limited to, Fab, F(ab')₂, Fd fragment, Fv, dAb, CDR, scFv, diabody and so on. Antigen portions such as Fab and F(ab')₂ fragments may be prepared from the full-length antibody by conventionally used techniques, e.g., papain or pepsin digestion of the whole antibody. Alternatively, an antibody and antigen-binding fragments thereof may be obtained by standard recombinant DNA techniques.

In the present invention, various genetic engineering and protein engineering procedures can be used to prepare antigen-binding fragments or antibody fragments which are portions of the monoclonal antibody, antibody-like molecules (e.g., low molecular antibody, genetically recombinant antibody, modified antibody), or a protein fused with the monoclonal antibody. More specifically, examples include, but are not limited to, H chain, L chain, Fv, Fab, Fab', F(ab')₂, scFv, sdFv, sc(Fv)₂, (scFv)₂, diabody, chimeric antibody, humanized antibody, human antibody, single chain antibody, multi-specific antibody (e.g., bispecific antibody) and so on. All of them fall within the anti-MCT5 antibody of the present invention as long as they are molecules having the ability to bind to an extracellular region of MCT5.

MCT5 recognized by the anti-MCT5 antibody of the present invention is a molecular marker which is highly expressed in cancer cells having high invasive capacity, but not in normal cells, in a population of cells to be detected. Thus, the antibody binds to highly malignant cancer cells.

In the context of the present invention, the term "cancer cells" is intended to mean a population of cells characterized by having high proliferative ability, having no limit on the number of cell divisions, and causing invasion and/or metastasis to surrounding tissues, when compared to normal cells.

In the context of the present invention, the term "cancer cells having high invasive capacity" is intended to mean cancer cells having the ability to invade a site remote from their initial location. For example, human breast cancer MCF7 cells have extremely low or no invasive capacity. On the other hand, MCF7-14 cells, which are MCF7 cells engineered to enhance their invasive capacity, have high invasive capacity and will invade from their transplanted site to other organs, and are therefore cells having high invasive capacity as their property (BMC Cancer (2010), 10, 414). Cells having high invasive capacity may be evaluated by invasive capacity assay using a Matrigel invasion chamber, etc. Matrigel mimics the basement membrane and allows these cells to be detected as a population of cells easy to permeate (invade) a Matrigel layer when they are seeded above the Matrigel layer and cultured.

In the context of the present invention, the term "normal cells" refers to cells having normal functions *in vivo* or in tissue activities. Such normal cells may include somatic stem cells, but are preferably mature cells.

In another embodiment of the present invention, the anti-MCT5 antibody of the present invention not only strongly binds to cancer cells having high invasive capacity, but is also capable of binding to cancer cells of one or more cancer types. These cancer types and cancer cells are preferably one or more cancer types of cellular or tissue origin (e.g., heart, brain, placenta, lung, liver, skeletal muscle, kidney, pancreas, spleen, thymus, prostate, testis, ovary, small intestine, leukocytes, colon, stomach, bone marrow, colorectum, and peripheral blood mononuclear cells), and are more preferably cancer cells from breast cancer and colorectal cancer.

In yet another embodiment of the present invention, the anti-MCT5 antibody of the present invention does not bind to normal cells. Preferably, the anti-MCT5 antibody of the present invention does not bind to normal cells in at least one or more of heart, brain, placenta, lung, skeletal muscle, kidney, spleen, thymus, prostate, testis, ovary, small intestine, leukocytes, colon, bone marrow, colorectum, and peripheral blood mononuclear cells, by way of example.

The anti-MCT5 antibody of the present invention is preferably an anti-MCT5 monoclonal antibody produced by the following hybridomas. Cell lines (hybridomas) producing the monoclonal antibody of the present invention may be exemplified by "Mouse-Mouse hybridoma IMI4C4a" (hereinafter also referred to as "IMI4C4a") and "Mouse-Mouse hybridoma IMI4C12a" (hereinafter also referred to as "IMI4C12a").

In yet another embodiment of the present invention, the anti-MCT5 antibody of the present invention is a chimeric antibody. The term "chimeric antibody" is intended to mean an antibody whose heavy and light chain constant regions are of human origin and whose heavy and light chain variable regions are of non-human (e.g., mouse) origin. As used herein, an antibody whose constant regions are of human origin and whose variable regions are of mouse origin, or an antigen-binding fragment thereof may also be referred to as a mouse-human chimeric antibody or an antigen-binding fragment thereof.

The anti-MCT5 antibody of the present invention having these features may be obtained by immunization with a partial protein of an extracellular domain of MCT5. MCT5 in databases is a 12-transmembrane protein, so that ICD4, which is an antigen of the antibody IMI4C4a, has been predicted to be an intracellular domain. However, as disclosed in Japanese Patent Application No. 2014-203162, the transmembrane number and/or extracellular domains of MCT5 are not in agreement with those in the databases, and at least ICD4 is exposed to the extracellular environment. Namely, to obtain a monoclonal antibody recognizing an extracellular domain of MCT5, a region serving as an extracellular domain should be identified and a monoclonal antibody against this region should be prepared. When an amino acid sequence comprising the thus newly predicted extracellular domain is used as an antigen to prepare a monoclonal antibody, the resulting monoclonal antibody may be used as an anti-MCT5 antibody.

In one embodiment of the present invention, the anti-MCT5 antibody of the present invention has the following features.

Since the three-dimensional structure of MCT5 was found to differ from that in the databases, the antibody of the present invention is a newly obtained antibody and is therefore capable of recognizing native MCT5 on the cell surface. On the other hand, a conventional monoclonal antibody (HPA046986) whose antigen is located near the epitope of the anti-MCT5 antibody IMI4C4a cannot recognize native MCT5 and therefore does not bind to MCT5 on the cell surface. In contrast, the recognition site of the antibody of the present invention binds to an extracellular domain of MCT5 and is therefore capable of binding to living cells. For this reason, the anti-MCT5 antibody of the present invention is effective as a therapeutic agent targeting MCT5-expressing cancer cells.

In addition, conventional antibodies are polyclonal antibodies and have been difficult to produce continuously as homogeneous antibodies, whereas the antibody of the present invention is a monoclonal antibody and hence can be mass-produced with high reproducibility. In terms of these features, the antibody of the present invention can be used for cancer treatment.

Moreover, the antibody against MCT5 or an antigen-binding fragment thereof is not limited only to the mouse monoclonal antibody against MCT5 produced by the above hybridoma IMI4C4a, and any other antibodies also fall within the anti-MCT5 antibody of the present invention as long as they bind to an epitope which is recognized by the monoclonal antibody produced from this hybridoma. As used herein, the term "epitope" refers to an epitope which is recognized by the monoclonal antibody produced from the above hybridoma (e.g., amino acid residues at positions 196 to 299 in the amino acid sequence of MCT5, or a partial region thereof). Further, such an epitope may be another region in MCT5, e.g., amino acid residues at positions 38 to 87 or a partial region thereof, or amino acid residues at positions 416 to 458 or a partial region thereof.

The antibody of the present invention may be a genetically recombinant antibody or an antigen-binding fragment thereof, which is prepared and expressed by recombination means. For example, the anti-MCT5 antibody of the present invention may be a chimeric antibody, a humanized antibody or a complete human antibody. The recombinant antibody of the present invention may be prepared by recombinant expression of heavy and light chains. For example, a mouse-human chimeric antibody may be prepared by isolating antibody genes from mouse cells producing an antibody against the MCT5 protein and causing recombination between its heavy chain (H chain) constant region and human IgG H chain constant region gene, followed by introduction into mouse myeloma cells. A humanized antibody may be prepared, for example, by grafting an antibody molecule of human origin with antigen-binding site genes isolated from mouse cells producing an antibody against the MCT5 protein. Likewise, a human antibody may be prepared by immunizing the MCT5 protein into mice whose immune system has been replaced with the human immune system. Moreover, a protein fused with a monoclonal antibody may be prepared using the antigen-binding variable region of the antibody and another protein through existing procedures for gene recombination. Alternatively, it may be prepared by crosslinking the monoclonal antibody and the protein via a crosslinker.

To express a genetically recombinant antibody, for example, recombinant expression vectors having nucleic acids encoding the heavy and light chains of this antibody are introduced into host cells, and the host cells carrying these vectors are cultured. The desired antibody can be collected from the cultured product of these host cells. These nucleic acids, recombinant expression vectors and host cells carrying the vectors, and a method for preparation of an antibody or an antigen-binding fragment thereof using these host cells are included in the present invention. In order that genes for antibody heavy and light chains are obtained and these nucleic acids are integrated into expression vectors and introduced into host cells, recombinant DNA techniques standard in the art (see, e.g., Sambrook et al., Molecular Cloning, Cold Spring Harbor, N.Y.) may be used for this purpose.

DNA fragments encoding VH and VL may further be engineered to encode, for example, Fab genes, scFv genes or full-length antibody genes by recombinant DNA techniques standard in the art.

Moreover, a nucleic acid (e.g., DNA) encoding VH may be converted into a full-length heavy chain gene when DNA encoding VH is expressibly linked to DNAs encoding heavy chain constant regions (CHI, CH2 and CH3). Nucleic acid sequences encoding heavy chain constant regions of human, mouse or other origin are known in the art.

A preferred example of the anti-MCT5 antibody of the present invention is an antibody in which the amino acid sequences of complementarity determining regions (CDRs) 1 to 3 in the heavy chain variable region (VH) comprise or consist of the amino acid sequences shown in SEQ ID NOs: 7, 8 and 9, respectively, and/or the amino acid sequences of complementarity determining regions (CDRs) 1 to 3 in the light chain variable region (VL) comprise or consist of the amino acid sequences shown in SEQ ID NOs: 12, 13 and 14, respectively. In another embodiment, a preferred example of the anti-MCT5 antibody of the present invention is an antibody in which the amino acid sequence of the heavy chain variable region (VH) comprises or consists of the amino acid sequence shown in SEQ ID NO: 6 (which is encoded by the nucleotide sequence shown in SEQ ID NO: 5), and/or the amino acid sequence of the light chain variable region (VL) comprises or consists of the amino acid sequence shown in SEQ ID NO: 11 (which is encoded by the nucleotide sequence shown in SEQ ID NO: 10).

H chain V region sequence
DNA sequence (SEQ ID NO: 5)

ATGAAATGCAGCTGGGTCATCTTCTTCCTGATGGCAGTGGTTACAGGGGTCA
ATTCAGAGGTTCAGCTGCAGCAGTCTGGGGCTGAGCTTGTGAGGCCAGGGG
CCTTAGTCAAGTTGTCCTGCAAAGCTTCTGGCTTCAACATTAAAGACTACTA
TATGTTCTGGGTGAAGCAGAGGCCTGAACAGGGCCTGGAGTGGATTGGATG
GATTGATCCTGAGAATGGTAATACTATATTTGACCCGAAGTTCCAGGGCAA
GGCCAGTATAACAGCAGACACATCCTCCAACACAGCCTACCTGCAGCTCAG
CAGCCTGACATCTGAGGACACTGCCGTCTATTACTGTGCTAGAATGATTACG
ACCTATTACTATGCTATGGACTTCTGGGGTCAAGGAACCTCAGTCACCGTCT
CCTCAGCC

Amino acid sequence (SEQ ID NO: 6)

MKCSWVIFFLMAVVTGVNSEVQLQQSGAELVRPGALVKLSCKASGFNIKDYY
MFWVKQRPEQGLEWIGWIDPENGNTIFDPKFQGKASITADTSSNTAYLQLSSLT
SEDTAVYYCARMITTYYYAMDFWGQGTSVTVSSA

H chain CDR regions
VH-CDR1: FNIKDYYMF (46-54) (SEQ ID NO: 7)
VH-CDR2: WIDPENGNTIFDPKFQGK (69-86) (SEQ ID NO: 8)
VH-CDR3: MITTYYYAMDFW (118-129) (SEQ ID NO: 9)

L chain V region sequence
DNA sequence (SEQ ID NO: 10)

ATGGCCTGGACTCCTCTCTTCTTCTTCTTTGTTCTTCATTGCTCAGGTTCTTTC
TCCCAACTTGTGCTCACTCAGTCATCTTCAGCCTCTTTCTCCCTGGGAGCCTC
AGCAAAACTCACGTGCACCTTGAGTAGTCAGCACAGTACGTACACCATTGA
ATGGTATCAGCAACAGCCACTCAAGCCTCCTAAGTATGTGATGGAGCTTAA
GAAAGATGGAAGCCACAGCACAGGTGATGGGATTCCTGATCGCTTCTCTGG
ATCCAGCTCTGGTGCTGATCGCTACCTTAGCGTTTCCAGCATCCAGCCTGAA
GATGAAGCAATATACATCTGTGGTGTGGGTTATACAATTAAGGAACAATTT
GTGTATGTTTTCGGCGGTGGAACCAAG

Amino acid sequence (SEQ ID NO: 11)

MAWTPLFFFFVLHCSGSFSQLVLTQSSSASFSLGASAKLTCTLSSQHSTYTIEWY
QQQPLKPPKYVMELKKDGSHSTGDGIPDRFSGSSSGADRYLSVSSIQPEDEAIYI
CGVGYTIKEQFVYVFGGGTK

L chain CDR regions
VL-CDR1: SQHSTYT (45-51) (SEQ ID NO: 12)
VL-CDR2: ELKKDGSHST (68-77) (SEQ ID NO: 13)
VL-CDR3: GYTIKEQ (114-120) (SEQ ID NO: 14)

When the nucleic acids encoding VH and VL are, for example, those of mouse origin and the nucleic acids encoding heavy and light chain constant regions are those of human origin, a mouse-human chimeric antibody or an antigen-binding fragment thereof can be obtained.

To express the antibody of the present invention or an antigen-binding fragment thereof, the partial or full-length heavy and light chain genes obtained as above are each inserted into an expression vector. The heavy and light chain genes are inserted into separate vectors or are both inserted into the same expression vector. These antibody genes may be inserted into an expression vector(s) in a standard manner. Moreover, such an expression vector may be allowed to encode a signal peptide which facilitates antibody chain secretion from host cells. Such a signal peptide may be an immunoglobulin signal peptide or may be a heterologous signal peptide. In addition, such an expression vector may further contain an additional regulatory sequence(s), and those skilled in the art would be able to select and introduce a regulatory sequence(s) into the expression vector on the basis of known techniques.

### Epitope for anti-MCT5 antibody

The epitope (antigenic determinant) for the anti-MCT5 antibody of the present invention is not limited in any way as long as it is at least a part of the antigen MCT5, but it is preferably at least a part of an extracellular region of MCT5, for example, at least a part of a region consisting of amino acids at positions 196 to 299 (SEQ ID NO: 15) in the amino acid sequence of MCT5 shown in SEQ ID NO: 2. Above all, preferred is at least a part of a region consisting of amino acids at positions 227 to 258 (SEQ ID NO: 16), and particularly preferred is at least a part of a region consisting of amino acids at positions 251 to 258 (SEQ ID NO: 4). The anti-MCT5 antibody recognizing such a region (binding to such a region) is, for example, very useful for use in detection and diagnosis of cancer as described later because of ensuring high detection sensitivity for MCT5 in biological samples.

ICD4 (SEQ ID NO: 15) 
RPIHIKSENNSGIKDKGSSLSAHGPEAHATETHCHETEESTIKDSTTQKAGLPSKN
LTVSQNQSEEFYNGPNRNRLLLKSDEESDKVISWSCKQLFDISLFRNP
 ICD4_227-258 (SEQ ID NO: 16)
THCHETEESTIKDSTTQKAGLPSKNLTVSQNQ

Moreover, MCT5 has six isoforms due to differences in mRNA splicing. More specifically, human MCT5 has isoforms as shown below: (i) isoform 1 (SEQ ID NO: 2) having the longest amino acid sequence which is a representative sequence of MCT5; (ii) a peptide (isoform 2) lacking amino acids at positions 74 to 121 of isoform 1; (iii) a peptide (isoform 3) lacking N-terminal 62 amino acids in isoform 1 and having a different amino acid sequence (SEQ ID NO: 18) at positions 63 to 73 from the corresponding amino acid sequence (SEQ ID NO: 17) of isoform 1; (iv) a peptide (isoform 4) lacking N-terminal 110 amino acids in isoform 1, having a different amino acid sequence (SEQ ID NO: 19) at positions 111 to 120 from the corresponding amino acid sequence of isoform 1 and having a different amino acid sequence (SEQ ID NO: 20) at position 446 to the C-terminal end from the corresponding amino acid sequence of isoform 1 (SEQ ID NO: 2); (v) a peptide (isoform 5) lacking the amino acid sequence at positions 176 to 343 of isoform 1; and (vi) a peptide (isoform 6) lacking N-terminal 62 amino acids in isoform 1, having a different amino acid sequence (SEQ ID NO: 18) at positions 63 to 73 from the corresponding amino acid sequence (SEQ ID NO: 17) of isoform 1 and lacking amino acids at positions 176 to 343 of isoform 1, etc.

Among these peptides, three isoforms, i.e., isoform 2, isoform 3 and isoform 4 are identical with isoform 1 of MCT5 in that they have in common a region consisting of amino acids at positions 196 to 299 in the amino acid sequence of isoform 1 of MCT5 shown in SEQ ID NO: 2. Thus, the antibody of the present invention allows detection of these three isoforms, in addition to isoform 1.

Likewise, an antibody prepared using an antigen comprising SEQ ID NO: 17 also allows detection of isoform 2 and isoform 5, in addition to isoform 1. Further, an antibody prepared using SEQ ID NO: 19 also allows detection of isoform 3, isoform 4 and isoform 6.

Moreover, the epitope (antigenic determinant) for the anti-MCT5 antibody of the present invention also includes at least a part of the corresponding region in MCT5 of other animal origin.

The antibody against MCT5 of the present invention includes an antibody binding to a site (e.g., epitope) to which the antibody binds, as exemplified by an antibody binding to a site to which an antibody produced by the hybridoma of the present invention binds.
Amino acid sequence at positions 63 to 73 of isoform 1 (SEQ ID NO: 17)
   GSIMSSLRFCA
Sequence (SEQ ID NO: 18) possessed by isoform 3 and isoform 6, which is different from the sequence of SEQ ID NO: 17
   MGMDDCDSFFP
Amino acid sequence (SEQ ID NO: 19) possessed by isoform 4, which is different from the sequence at positions 111 to 120 of isoform 1
   MGMDDCDSFF
Amino acid sequence (SEQ ID NO: 20) possessed by isoform 4, which is different from the sequence at position 446 to the C-terminal end of isoform 1
   EIIPSFQAGYMIIPRHTMALSTSLAYAISSLQFPFFLYHWPKDGKTV

### Preparation of genetically recombinant antibodies

A preferred embodiment of the antibody of the present invention may be a genetically recombinant antibody. Examples of a genetically recombinant antibody include, but are not limited to, a chimeric antibody, a reshaped human antibody and a humanized antibody, etc.

A chimeric antibody (i.e., a humanized chimeric antibody) is an antibody in which antibody variable regions of mouse origin are linked (conjugated) to constant regions of human origin (see, e.g., Proc. Natl. Acad. Sci. U.S.A. 81, 6851-6855 (1984)). For preparation of a chimeric antibody, gene recombination technology may be used for its construction such that the thus linked antibody is obtained.

In this regard, the antibody variable regions of mouse origin are preferably composed of a heavy chain variable region which comprises or consists of, for example, the amino acid sequence shown in SEQ ID NO: 6 and a light chain variable region which comprises or consists of, for example, the amino acid sequence shown in SEQ ID NO: 11.

A preferred mouse-human chimeric antibody comprises or consists of the amino acid sequence shown in SEQ ID NO: 29 for its heavy chain variable region linked to a constant region of human origin and the amino acid sequence shown in SEQ ID NO: 32 for its light chain variable region linked to a constant region of human origin.

For preparation of a reshaped human antibody, it is possible to use a process referred to as so-called CDR grafting (CDR transplantation). CDR grafting is a technique to prepare reshaped variable regions whose framework regions (FRs) are of human origin and whose CDRs are of mouse origin by transplanting complementarity determining regions (CDRs) from mouse antibody variable regions to human variable regions. Next, these humanized reshaped human variable regions are linked to human constant regions. Procedures for preparation of such a reshaped human antibody are well known in the art (see, e.g., Nature, 321, 522-525 (1986); J. Mol. Biol., 196, 901-917 (1987); Queen C et al., Proc. Natl. Acad. Sci. USA, 86: 10029-10033 (1989); Japanese Patent No. 2828340). In this regard, the amino acid sequences of CDRs of mouse origin which can be used for the reshaped human anti-MCT5 antibody of the present invention are preferably, but not limited to, the amino acid sequences shown in SEQ ID NOs: 7, 8 and 9 for heavy chain variable region CDRs 1 to 3, respectively, and the amino acid sequences shown in SEQ ID NOs: 12, 13 and 14 for light chain variable region CDRs 1 to 3, respectively, by way of example.

A human antibody (complete human antibody) is generally an antibody in which hyper variable regions serving as antigen-binding sites in its variable regions (V regions), the other regions in its V regions and its constant regions have the same structures as those of human antibody. Techniques for human antibody preparation are also known, and in the case of gene sequences common to humans, an approach has been established for their preparation by genetic engineering procedures. Such a human antibody may be obtained, for example, by using human antibody-producing mice which have human chromosome fragments comprising genes for human antibody heavy chain (H chain) and light chain (L chain) (see, e.g., Tomizuka, K. et al., Nature Genetics, (1977) 16, 133-143; Kuroiwa, Y. et al., Nuc. Acids Res., (1998) 26, 3447-3448; Yoshida, H. et al., Animal Cell Technology: Basic and Applied Aspects, (1999) 10, 69-73 (Kitagawa, Y., Matuda, T. and Iijima, S. eds.), Kluwer Academic Publishers; Tomizuka, K. et al., Proc. Natl. Acad. Sci. USA, (2000) 97, 722-727) or by obtaining a phage display-derived human antibody selected from human antibody libraries (see, e.g., Wormstone, I. M. et al, Investigative Ophthalmology & Visual Science., (2002) 43 (7), 2301-8; Carmen, S. et al., Briefings in Functional Genomics and Proteomics, (2002) 1 (2), 189-203; Siriwardena, D. et al., Opthalmology, (2002) 109 (3), 427-431).

Alternatively, in the present invention, a hybridoma or DNA or RNA extracted from this hybridoma may be used as a starting material to prepare a chimeric antibody, a reshaped human antibody or a humanized antibody in accordance with the well-known approaches mentioned above.

Further, a protein fused with the antibody of the present invention may be prepared from the antibody variable regions and any other protein by known gene recombination techniques. Alternatively, such a fusion protein may be prepared by crosslinking the monoclonal antibody with any other protein using a crosslinker.

### Preparation of antibody fragments

A fragment of the antibody against MCT5 for use in the present invention specifically binds to MCT5.

A fragment of the antibody is intended to mean a partial region of the antibody of the present invention, and examples include Fab, Fab', F(ab')₂, Fv, diabody (dibodies), dsFv, scFv (single chain Fv) and so on. The above antibody fragments may be obtained by cleaving the antibody of the present invention with various proteases depending on the intended purpose.

For example, Fab may be obtained by treating an antibody molecule with papain, while F(ab')₂ may be obtained by treating an antibody molecule with pepsin. Likewise, Fab' may be obtained by cleaving the disulfide bonds in the hinge region of the above F(ab')₂.

In the case of scFv, cDNAs encoding antibody heavy chain variable region (H chain V region) and light chain variable region (L chain V region) may be obtained to construct DNA encoding scFv. This DNA may be inserted into an expression vector, and the resulting expression vector may be introduced into a host organism to cause expression, whereby scFv may be prepared.

In the case of diabody, cDNAs encoding antibody H chain V region and L chain V region may be obtained to construct DNA encoding scFv such that the amino acid sequence of a peptide linker has a length of 8 residues or less. This DNA may be inserted into an expression vector, and the resulting expression vector may be introduced into a host organism to cause expression, whereby diabody may be prepared.

In the case of dsFv, cDNAs encoding antibody H chain V region and L chain V region may be obtained to construct DNA encoding dsFv. This DNA may be inserted into an expression vector, and the resulting expression vector may be introduced into a host organism to cause expression, whereby dsFv may be prepared.

In the present invention, the nucleotide sequence of DNA encoding the heavy chain variable region may be exemplified by those comprising or consisting of the nucleotide sequence shown in SEQ ID NO: 5, while the nucleotide sequence of DNA encoding the light chain variable region may be exemplified by those comprising or consisting of the nucleotide sequence shown in SEQ ID NO: 10.

Moreover, specific examples of the antibody fragment of the present invention include, but are not limited to, antibody fragments comprising the amino acid sequences shown in SEQ ID NOs: 7, 8 and 9 for the amino acid sequences of VH CDRs 1 to 3, respectively, and/or comprising the amino acid sequences shown in SEQ ID NOs: 12, 13 and 14 for the amino acid sequences of VL CDRs 1 to 3, respectively. Further examples include antibody fragments comprising the amino acid sequence shown in SEQ ID NO: 6 for VH, and/or comprising the amino acid sequence shown in SEQ ID NO: 11 for VL.

A CDR-containing antibody fragment (peptide) is configured to comprise at least one or more regions of VH or VL CDRs (CDRs 1 to 3). In the case of an antibody fragment containing a plurality of CDRs, these CDRs may be linked directly or via an appropriate peptide linker. For preparation of a CDR-containing antibody fragment, DNA encoding antibody VH and VL CDRs may be constructed, and this DNA may be inserted into an expression vector for prokaryotic organisms or an expression vector for eukaryotic organisms, and the resulting expression vector may be introduced into a prokaryotic organism or a eukaryotic organism to cause expression. Alternatively, a CDR-containing peptide may also be prepared by chemical synthesis such as Fmoc method (fluorenylmethyloxycarbonyl method) and tBoc method (t-butyloxycarbonyl method).

The nucleotide sequences encoding VH CDRs 1 to 3 are preferably the nucleotide sequences shown in SEQ ID NOs: 21, 22 and 23, respectively, by way of example, while the nucleotide sequences encoding VL CDRs 1 to 3 are preferably the nucleotide sequences shown in SEQ ID NOs: 24, 25 and 26, respectively, by way of example.
VH-CDR1 nucleotide sequence (SEQ ID NO: 21)
   TTCAACATTAAAGACTACTATATGTTC
VH-CDR2 nucleotide sequence (SEQ ID NO: 22) 
TGGATTGATCCTGAGAATGGTAATACTATATTTGACCCGAAGTTCCAGGGCA
AG
VH-CDR3 nucleotide sequence (SEQ ID NO: 23)
   ATGATTACGACCTATTACTATGCTATGGACTTCTGG
VL-CDR1 nucleotide sequence (SEQ ID NO: 24)
   AGTCAGCACAGTACGTACACC
VL-CDR2 nucleotide sequence (SEQ ID NO: 25)
   GAGCTTAAGAAAGATGGAAGCCACAGCACA
VL-CDR3 nucleotide sequence (SEQ ID NO: 26)
   GGTTATACAATTAAGGAACAA

Mouse-human chimeric antibody H chain nucleotide sequence (SEQ ID NO: 27) derived from the antibody IMI4C4a

ATGAAATGCAGCTGGGTCATCTTCTTCCTGATGGCAGTGGTTACAGGGGTCA
ATTCAGAGGTTCAGCTGCAGCAGTCTGGGGCTGAGCTTGTGAGGCCAGGGG
CCTTAGTCAAGTTGTCCTGCAAAGCTTCTGGCTTCAACATTAAAGACTACTA
TATGTTCTGGGTGAAGCAGAGGCCTGAACAGGGCCTGGAGTGGATTGGATG
GATTGATCCTGAGAATGGTAATACTATATTTGACCCGAAGTTCCAGGGCAA
GGCCAGTATAACAGCAGACACATCCTCCAACACAGCCTACCTGCAGCTCAG
CAGCCTGACATCTGAGGACACTGCCGTCTATTACTGTGCTAGAATGATTACG
ACCTATTACTATGCTATGGACTTCTGGGGTCAAGGAACCTCAGTCACCGTCT
CCGCAGCTAGCACCAAGGGCCCATCGGTCTTCCCCCTGGCACCCTCCTCCAA
GAGCACCTCTGGGGGCACAGCGGCCCTGGGCTGCCTGGTCAAGGACTACTT
CCCCGAACCGGTGACGGTGTCGTGGAACTCAGGCGCCCTGACCAGCGGCGT
GCACACCTTCCCGGCTGTCCTACAGTCCTCAGGACTCTACTCCCTCAGCAGC
GTGGTGACCGTGCCCTCCAGCAGCTTGGGCACCCAGACCTACATCTGCAAC
GTGAATCACAAGCCCAGCAACACCAAGGTGGACAAGAAAGTTGAGCCCAA
ATCTTGTGACAAAACTCACACATGCCCACCGTGCCCAGCACCTGAACTCCTG
GGGGGACCGTCAGTCTTCCTCTTCCCCCCAAAACCCAAGGACACCCTCATGA
TCTCCCGGACCCCTGAGGTCACATGCGTGGTGGTGGACGTGAGCCACGAAG
ACCCTGAGGTCAAGTTCAACTGGTACGTGGACGGCGTGGAGGTGCATAATG

CCAAGACAAAGCCGCGGGAGGAGCAGTACAACAGCACGTACCGTGTGGTC
AGCGTCCTCACCGTCCTGCACCAGGACTGGCTGAATGGCAAGGAGTACAAG
TGCAAGGTCTCCAACAAAGCCCTCCCAGCCCCCATCGAGAAAACCATCTCC
AAAGCCAAAGGGCAGCCCCGAGAACCACAGGTGTACACCCTGCCCCCATCC
CGGGATGAGCTGACCAAGAACCAGGTCAGCCTGACCTGCCTGGTCAAAGGC
TTCTATCCCAGCGACATCGCCGTGGAGTGGGAGAGCAATGGGCAGCCGGAG
AACAACTACAAGACCACGCCTCCCGTGCTGGACTCCGACGGCTCCTTCTTCC
TCTACAGCAAGCTCACCGTGGACAAGAGCAGGTGGCAGCAGGGGAACGTCT[?]
TCTCATGCTCCGTGATGCATGAGGCTCTGCACAACCACTACACGCAGAAGA
GCCTCTCCCTGTCTCCGGGTAAATGA

Mouse-human chimeric antibody H chain amino acid sequence (SEQ ID NO: 28) derived from the antibody IMI4C4a

MKCSWVIFFLMAVVTGVNSEVQLQQSGAELVRPGALVKLSCKASGFNIKDYY
MFWVKQRPEQGLEWIGWIDPENGNTIFDPKFQGKASITADTSSNTAYLQLSSLT
SEDTAVYYCARMITTYYYAMDFWGQGTSVTVSAASTKGPSVFPLAPSSKSTSG
GTAALGCLVKDYFPEPVTVSWNSGALTSGVHTFPAVLQSSGLYSLSSVVTVPSS
SLGTQTYICNVNHKPSNTKVDKKVEPKSCDKTHTCPPCPAPELLGGPSVFLFPPK
PKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTL
PPSRDELTKNQVSLTCLVKGFYPSDIAVEWESNGQPENNYKTTPPVLDSDGSFF
LYSKLTVDKSRWQQGNVFSCSVMHEALHNHYTQKSLSLSPGK*

Mouse-human chimeric antibody L chain nucleotide sequence (SEQ ID NO: 29) derived from the antibody IMI4C4a

ATGGCCTGGACTCCTCTCTTCTTCTTCTTTGTTCTTCATTGCTCAGGTTCTTTC
TCCCAACTTGTGCTCACTCAGTCATCTTCAGCCTCTTTCTCCCTGGGAGCCTC
AGCAAAACTCACGTGCACCTTGAGTAGTCAGCACAGTACGTACACCATTGA
ATGGTATCAGCAACAGCCACTCAAGCCTCCTAAGTATGTGATGGAGCTTAA
GAAAGATGGAAGCCACAGCACAGGTGATGGGATTCCTGATCGCTTCTCTGG
ATCCAGCTCTGGTGCTGATCGCTACCTTAGCGTTTCCAGCATCCAGCCTGAA
GATGAAGCAATATACATCTGTGGTGTGGGTTATACAATTAAGGAACAATTT
GTGTATGTTTTCGGCGGTGGAACCAAGGTCACTGTCCTAGGTCAGCCCAAG
GCTGCCCCCTCGGTCACTCTGTTCCCGCCCTCCTCTGAGGAGCTTCAAGCCA
ACAAGGCCACACTGGTGTGTCTCATAAGTGACTTCTACCCGGGAGCCGTGA
CAGTGGCCTGGAAGGCAGATAGCAGCCCCGTCAAGGCGGGAGTGGAGACC
ACCACACCCTCCAAACAAAGCAACAACAAGTACGCGGCCAGCAGCTATCTG
AGCCTGACGCCTGAGCAGTGGAAGTCCCACAGAAGCTACAGCTGCCAGGTC
ACGCATGAAGGGAGCACCGTGGAGAAGACAGTGGCCCCTACAGAATGTTCA
TAG

Mouse-human chimeric antibody L chain amino acid sequence (SEQ ID NO: 30) derived from the antibody IMI4C4a

MAWTPLFFFFVLHCSGSFSQLVLTQSSSASFSLGASAKLTCTLSSQHSTYTIEWY
QQQPLKPPKYVMELKKDGSHSTGDGIPDRFSGSSSGADRYLSVSSIQPEDEAIYI
CGVGYTIKEQFVYVFGGGTKVTVLGQPKAAPSVTLFPPSSEELQANKATLVCLI
SDFYPGAVTVAWKADSSPVKAGVETTTPSKQSNNKYAASSYLSLTPEQWKSHR
SYSCQVTHEGSTVEKTVAPTECS*

### Binding affinity

The binding affinity can be determined from the binding constant (KA) and the dissociation constant (KD). The affinity equilibrium constant (K) is expressed as the KA/KD ratio. The binding affinity may be detected in the following manner.

The antibody of the present invention has a dissociation constant (KD) of at least 1 × 10⁻¹⁰ M and has an affinity which is, for example, 2- to 5-fold, 5- to 10-fold, 10- to 100-fold, 100- to 1000-fold or 1000- to 10,000-fold higher than this dissociation constant. More specifically, the dissociation constant (KD) of the antibody of the present invention in relation to MCT5 binding affinity is 1 × 10⁻¹⁰ M, 5 × 10⁻¹¹ M, 1 × 10⁻¹¹ M, 5 × 10⁻¹² M,1 × 10⁻¹² M, 5 × 10⁻¹³ M,1 × 10⁻¹³ M, 5 × 10⁻¹⁴ M,1 × 10⁻¹⁴ M, 5 × 10⁻¹⁵ M or 1 × 10⁻¹⁵ M, and is preferably 1 × 10⁻¹⁰ M to 1 × 10⁻¹³ M. Alternatively, the antibody of the present invention may have a value lower than these KD values and a higher affinity.

In this regard, if the dissociation constant (KD) of an antibody to be measured for its affinity is within about 1- to 100-fold of the KD of the antibody of the present invention, this antibody is regarded as being substantially the same as the antibody of the present invention and therefore falls within the present invention.

The binding constant (KA) and the dissociation constant (KD) may be measured by surface plasmon resonance (SPR), and any known instrument and method which allow real-time detection and monitoring of the binding rate may be used for this purpose (e.g., Biacore® T200 (GE Healthcare), ProteON XPR36 (Bio-Rad)).

### Reagent for cancer treatment (anticancer agent)

Mammalian cells preferred to express the recombinant antibody of the present invention include Chinese hamster ovary cells (CHO cells), COS cells, 293 cells, HeLa cells, 3T3 cells and so on. Recombinant expression vectors encoding antibody heavy and light chains are introduced into preferred host cells by any known gene transfer techniques. The resulting transformant is cultured, and a desired antibody or an antigen-binding fragment thereof is collected from the cultured product. The antibody or antigen-binding fragment may be purified by any known purification techniques.

In another embodiment of the present invention, the anti-MCT5 antibody of the present invention is a chimeric antibody. The term "chimeric antibody" is intended to mean an antibody whose heavy and light chain constant regions are of human origin and whose heavy and light chain variable regions are of non-human (e.g., mouse) origin. As used herein, an antibody whose constant regions are of human origin and whose variable regions are of mouse origin, or an antigen-binding fragment thereof may also be referred to as a human-mouse chimeric antibody or an antigen-binding fragment thereof.

### Antibody conjugate of the present invention

In another embodiment of the present invention, the monoclonal antibody of the present invention (including an antigen-binding fragment thereof) may be conjugated with at least one chemotherapeutic agent (including an anticancer agent) or at least one toxin or radioisotope, and such a conjugate (also referred to as the antibody conjugate of the present invention) also falls within the scope of the present invention.

For example, in yet another embodiment of the present invention, there is provided an antibody conjugate comprising the monoclonal antibody of the present invention and a radioisotope, wherein the monoclonal antibody of the present invention forms a conjugate with a detectable radioisotope. Such a detectable radioisotope may be, for example, ³H, ¹⁴C, ³⁵S, ⁹⁰Y, ⁹⁹Tc, ¹¹¹In, ¹²⁵I, ¹³¹I, ¹⁷⁷Lu, ¹⁶⁶Ho or ¹⁵³Sm.

For example, in yet another embodiment of the present invention, there is provided an antibody conjugate comprising the monoclonal antibody of the present invention and an anticancer agent or a toxin, wherein the monoclonal antibody of the present invention forms a conjugate with the anticancer agent or toxin. The above chemotherapeutic agent is preferably an anticancer agent. Likewise, the toxin is preferably saponin, DM1 (N2'-deacetyl-N2'-(3-mercapto-1-oxopropyl)-maytansine), DM4 (N2'-deacetyl-N2'-(4-mercapto-4-methyl-1-oxopentyl)-maytansine), MMAE (monomethyl auristatin E) or SN38.

"Chemotherapeutic agents" are chemical compounds useful in cancer treatment, regardless of their mechanism of action during cancer chemotherapy. The types of chemotherapeutic agents include, but are not limited to, alkylating agents, antimetabolites, spindle poison plant alkaloids, cytotoxic/antitumor antibiotics, topoisomerase inhibitors, antibodies, photosensitizers and kinase inhibitors. Chemotherapeutic agents include not only compounds used for "targeting therapy" but also compounds used for conventional chemotherapy. Examples of chemotherapeutic agents include erlotinib (TARCEVA®, Genentech/OSI Pharm.), docetaxel (TAXOTERE®, Sanofi-Aventis), 5-FU (fluorouracil, 5-fluorouracil, CAS No. 51-21-8), gemcitabine (GEMZAR®, Lilly), PD-0325901 (CAS No. 391210-10-9, Pfizer), cisplatin (cis-diamine, dichloroplatinum(II), CAS No. 15,663-27-1), carboplatin (CAS No. 41575-94-4), paclitaxel (TAXOL®, Bristol-Myers Squibb Oncology, Princeton, N.J.), trastuzumab (HERCEPTIN®, Genentech), temozolomide (4-methyl-5-oxo-2,3,4,6,8-pentazabicyclo[4.3.0]nona-2,7,9-triene-9-carboxamide, CAS No. 85622-93-1, TEMODAR®, TEMODAL®, Schering Plough), tamoxifen ((Z)-2-[4-(1,2-diphenylbut-1-enyl)phenoxy]-N,N-dimethyl-ethaneamide, NOLVADEX®, ISTUBAL®, VALODEX®), and doxorubicin (ADRIAMYCIN®), Akti-1/2, HPPD and rapamycin.

Other examples of chemotherapeutic agents include oxaliplatin (ELOXATIN®, Sanofi), bortezomib (VELCADE®, Millennium Pharm.), sutent (SUNITINIB®, SU11248, Pfizer), letrozole (FEMARA®, Novartis), imatinib mesylate (GLEEVEC®, Novartis), XL-518 (Mek inhibitor, Exelixis, WO2007/044515), ARRY-886 (Mek inhibitor, AZD6244, Array BioPharma, Astra Zeneca), SF-1126 (PI3K inhibitor, Semafore Pharmaceuticals), BEZ-235 (PI3K inhibitor, Novartis), XL-147 (PI3K inhibitor, Exelixis), PTK787/ZK 222584 (Novartis), fulvestrant (FASLODEX®, AstraZeneca), leucovorin (folinic acid), rapamycin (sirolimus, RAPAMUNE®, Wyeth), lapatinib (TYKERB®, GSK572016, Glaxo Smith Kline), lonafarnib (SARASAR TM, SCH 66336, Schering Plough), sorafenib (NEXAVAR®, BAY43-9006, Bayer Labs), gefitinib (IRESSA®, AstraZeneca), irinotecan (CAMPTOSAR®, CPT-11, Pfizer), tipifarnib (ZARNESTRA TM, Johnson & Johnson), ABRAXANE TM (cremophor-free), an albumin-modified nanoparticle formulation of paclitaxel (American Pharmaceutical Partners, Schaumberg, II), vandetanib (rINN, ZD6474, ZACTIMA®, AstraZeneca), chlorambucil, AG1478, AG1571 (SU5271; Sugen), temsirolimus (TORISEL®, Wyeth), pazopanib (GlaxoSmithKline), canfosfamide (TELCYTA®, Telik), thiotepa, and cyclophosphamide (CYTOXAN®, NEOSAR®); alkyl sulfonates such as busulphan, improsulfan and piposulfan; aziridines such as benzodopa, carboquone, meturedopa, and uredopa; ethyleneimines and methylamelamines including altretamine, triethylenemelamine, triethylenephosphoramide, triethylenethiophosphaoramide and trimethylolomelamine; acetogenins (particularly bullatacin and bullatacinone); camptothecin (including the synthetic analog topotecan); bryostatin; callystatin; CC-1065 (including its adozelesin, carzelesin and bizelesin synthetic analogs); cryptophycins (particularly cryptophycin 1 and cryptophycin 8); dolastatin; duocarmycin (including the synthetic analogs, KW-2189 and CBI-TM1); eleutherobin; pancratistatin; sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide and uracil mustard; nitrosureas such as carmustine, chlorozotocin, fotemustine, lomustine, nimustine and ranimustine; antibiotics such as enediyne antibiotics (e.g., calicheamicin, calicheamicin gamma 1I and calicheamicin omega I1 (see, e.g., Agnew Chem Intl. Ed. Engl., 33:183-186 (1994)); dynemicin, dynemicinA; bisphosphonates such as clodronate; esperamicin; as well as neocarzinostatin chromophore and related chromoprotein enediyne antibiotic chromophores), aclacinomysins, actinomycin, authramycin, azaserine, bleomycins, cactinomycin, carabicin, carminomycin, carzinophilin, chromomycin, dactinomycin, daunorubicin, detorubicin, 6-diazo-5-oxo-L-norleucine, morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogs such as denopterin, methotrexate, pteropterin and trimetrexate; purine analogs such as fludarabine, 6-mercaptopurine, thiamiprine and thioguanine; pyrimidine analogs such as ancitabine, azacitidine, 6-azauridine, carmofur, cytarabine, dideoxyuridine, doxifluridine, enocitabine and floxuridine; androgens such as calusterone, dromostanolone propionate, epitiostanol, mepitiostane, testolactone; antiadrenal agents such as aminoglutethimide, mitotane and trilostane; folic acid replenishers such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; epothilone; etoglucid; gallium nitrate; hydroxyurea; lentinan; lonidamine; maytansinoids such as maytansine and ansamitocin; mitoguazone; mitoxantrone; mopidamol; nitracrine; pentostatin; phenamet; pirarubicin; podophyllinic acid; 2-ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (T-2 toxin, verracurin A, roridin A and anguidine); urethane; vindesine; dacarbazine; mannomustine; mitobronitol; mitolactol; pipobroman; gacytosine; arabinoside ("Ara-C"); cyclophosphamide; thiotepa; 6-thioguanine; mercaptopurine; methotrexate; platinum analogs such as cisplatin and carboplatin; vinblastine; etoposide (VP-16); mitoxantrone; vincristine; vinorelbine (NAVELBINE®); navelbine; novantrone; teniposide; edatrexate; daunomycin; aminopterin; capecitabine (XELODA®, Roche); ibandronate; CPT-11; topoisomerase inhibitor RFS2000; difluoromethylornithine (DMFO); retinoids such as retinoic acid; as well as pharmaceutically acceptable salts, acids and derivatives of the above members.

Also included in the definition of "chemotherapeutic agents" are: (i) anti-hormonal agents that act to regulate or inhibit the action of hormones on tumors, e.g., anti-estrogens and selective estrogen receptor modulators (SERMs), as exemplified by tamoxifen (NOLVADEX® tamoxifen citrate), raloxifene, droloxifene, 4-hydroxytamoxifen, trioxifene, keoxifene, LY117018, onapristone and FARESTON® (toremifene citrate); (ii) aromatase inhibitors that inhibit the enzyme aromatase, which regulates estrogen production in the adrenal glands, as exemplified by 4(5)-imidazoles, aminoglutethimide, MEGASE® (megestrol acetate), AROMASIN® (exemestane, Pfizer), formestanie, fadrozole, RIVISOR® (vorozole), FEMARA® (letrozole, Novartis) and ARIMIDEX® (anastrozole, AstraZeneca); (iii) anti-androgens, as exemplified by flutamide, nilutamide, bicalutamide, leuprolide, and goserelin; as well as troxacitabine (a 1,3-dioxolane nucleoside cytosine analog); (iv) protein kinase inhibitors, as exemplified by MEK inhibitors (WO2007/044515); (v) lipid kinase inhibitors; (vi) antisense oligonucleotides, particularly those which inhibit the expression of genes in signaling pathways implicated in aberrant cell proliferation, e.g., PKC-α, Raf and H-Ras, as exemplified by oblimersen (GENASENSE®, Genta Inc.); (vii) ribozymes, as exemplified by VEGF expression inhibitors (e.g., ANGIOZYME®) and HER2 expression inhibitors; (viii) gene therapy vaccines, as exemplified by vaccines such as ALLOVECTIN®, LEUVECTIN® and VAXID®; PROLEUKIN® rIL-2; topoisomerase 1 inhibitors such as LURTOTECAN®; ABARELIX® rmRH; (ix) anti-angiogenic agents, as exemplified by bevacizumab (AVASTIN®, Genentech); as well as pharmaceutically acceptable salts, acids or derivatives of the above members. Also included in the definition of "chemotherapeutic agents" are therapeutic antibodies, as exemplified by alemtuzumab (Campath), bevacizumab (Avastin®, Genentech); cetuximab (ERBITUX®, Imclone); panitumumab (VECTIBIX®, Amgen), rituximab (RITUXAN®, Genentech/Biogen Idec), pertuzumab (OMNITARG TM, 2C4, Genentech), trastuzumab (HERCEPTIN®, Genentech), tositumomab (Bexxar, Corixia), and the antibody-drug conjugate, gemutuzumab ozogamicin (MYLOTARG®, Wyeth).

Humanized monoclonal antibodies with therapeutic potential as chemotherapeutic agents in combination with the anti-MCT5 monoclonal antibody include alemtuzumab, apolizumab, aselizumab, atlizumab, bapineuzumab, bevacizumab, bivatuzumab mertansine, cantuzumab mertansine, cedelizumab, certolizumab pegol, cidfusituzumab, cidtuzumab, daclizumab, eculizumab, efalizumab, epratuzumab, erlizumab, felvizumab, fontolizumab, gemutuzumab ozogamicin, inotuzumab ozogamicin, ipilimumab, labetuzumab, lintuzumab, matuzumab, mepolizumab, motavizumab, motovizumab, natalizumab, nimotuzumab, nolovizumab, numavizumab, ocrelizumab, omalizumab, palivizumab, pascolizumab, pecfusituzumab, pectuzumab, pertuzumab, pexelizumab, ralivizumab, ranibizumab, reslivizumab, reslizumab, resyvizumab, rovelizumab, ruplizumab, sibrotuzumab, siplizumab, sontuzumab, tacatuzumab tetraxetan, tadocizumab, talizumab, tefibazumab, tocilizumab, toralizumab, trastuzumab, tucotuzumab celmoleukin, tucusituzumab, umavizumab, urtoxazumab and visilizumab.

A "metabolite" is a product produced through metabolism in the body of a specific compound or salt thereof. Metabolites of a compound may be identified using routine techniques known in the art, and their activity may be determined using tests such as those described herein. Such products may result from, e.g., oxidation, reduction, hydrolysis, amidation, deamidation, esterification, deesterification, enzymatic cleavage and the like, of the administered compound. Thus, the present invention includes metabolites of the compound of the present invention, including compounds produced by a method which involves contacting the compound of the present invention with a mammal for a period of time sufficient to yield metabolites thereof.

The monoclonal antibody of the present invention may be directly or indirectly linked (conjugated) with a chemotherapeutic agent (including an anticancer agent), a toxin or a radioisotope. Indirect conjugation includes conjugation via a linker and conjugation via an antibody or an antibody fragment, which binds to the monoclonal antibody of the present invention. For example, an antibody conjugate formed by binding between an antibody conjugated with a labeling substance (e.g., a fluorescent label) and the monoclonal antibody of the present invention also falls within the present invention.

Further, the monoclonal antibody of the present invention desirably comprises an antigen-binding region specific for effector cells having tumoricidal activity or tumoristatic activity, as exemplified by natural killer cells (NK cells) and macrophages, etc.

As used herein, the term "tumoricidal activity" is intended to mean the activity to destroy or kill tumor cells, while the term "tumoristatic activity" is intended to mean the activity to reduce the number of tumor cells or the activity to reduce the proliferation rate of tumor cells.

Once the monoclonal antibody of the present invention comprising an antigen-binding region specific for effector cells has bound to cancer cells, the effector cells will bind to the antibody, whereby the cancer cells can be killed by antibody-dependent cellular cytotoxicity (ADCC).

Likewise, once the monoclonal antibody of the present invention comprising such a region has bound to cancer cells, the complement system will be activated, whereby the cancer cells can be killed by CDC activity (Complement-Dependent Cytotoxicity).

### Pharmaceutical composition of the present invention

In the present invention, there is provided a pharmaceutical composition comprising the anti-MCT5 antibody of the present invention (including an antigen-binding fragment thereof). The anti-MCT5 antibody of the present invention recognizes MCT5 expressed in cancer cells. The pharmaceutical composition of the present invention comprising the anti-MCT5 antibody of the present invention can be used to kill MCT5-expressing cancer cells. Namely, the pharmaceutical composition of the present invention is useful as a pharmaceutical composition for cancer treatment, preferably as a pharmaceutical composition for use in treatment of colorectal cancer. Alternatively, the pharmaceutical composition of the present invention may also be used as a therapeutic agent for cancer.

The monoclonal antibody of the present invention may be conjugated with a chemotherapeutic agent, a toxin or a radioisotope. Thus, in another embodiment of the present invention, there is provided a pharmaceutical composition comprising the antibody conjugate of the present invention.

Since the monoclonal antibody of the present invention recognizes MCT5 expressed in cancer cells, the pharmaceutical composition of the present invention comprising the monoclonal antibody of the present invention (including the antibody conjugate of the present invention) is useful as a pharmaceutical composition for cancer treatment, preferably as a pharmaceutical composition for use in treatment of colorectal cancer.

Moreover, the pharmaceutical composition of the present invention may also be used as a therapeutic agent for cancer. The therapeutic agent for cancer intended in the present invention comprises the anti-MCT5 antibody alone or comprises the anti-MCT5 antibody optionally linked to a pharmaceutically acceptable anticancer agent or carrier, as appropriate. Alternatively, the therapeutic agent for cancer intended in the present invention may be used in combination with other modes of therapy.

Moreover, the monoclonal antibody of the present invention recognizes MCT5 expressed in cancer cells and is taken up into the cells through the internalization mechanism after binding to MCT5 on the cell membrane. Thus, the pharmaceutical composition of the present invention can be used to kill MCT5-expressing cancer cells. In particular, in the case of comprising an antibody conjugate comprising a chemotherapeutic agent and the monoclonal antibody of the present invention, the chemotherapeutic agent conjugated with the antibody can also be taken up into cancer cells, so that the pharmaceutical composition of the present invention is useful for killing cancer cells in a cancer patient.

In the present invention, cancer treatment includes killing cancer cells, reducing the size of cancer, suppressing or arresting the rate of cancer growth, or suppressing or arresting the progression of cancer, by way of example.

The pharmaceutical composition of the present invention may be formulated as a pharmaceutical composition comprising the antibody of the present invention in combination with a chemotherapeutic agent, a toxin or a radioisotope. As an anticancer agent to be used in combination, the same chemotherapeutic agent as that contained in the pharmaceutical composition of the present invention may be used, or alternatively, a different chemotherapeutic agent may be used. The dose and mode of administration of such an anticancer agent may be determined as appropriate by those skilled in the art.

The pharmaceutical composition of the present invention may be used to treat any type of cancer where MCT5 is expressed, and examples include malignant melanoma, malignant lymphoma, digestive organ cancer, lung cancer, esophageal cancer, gastric cancer, colorectal cancer, rectal cancer, colon cancer, urinary tract tumor, gallbladder cancer, bile duct cancer, biliary tract cancer, breast cancer, liver cancer, pancreatic cancer, testicular tumor, maxillary cancer, tongue cancer, lip cancer, oral cancer, pharyngeal cancer, laryngeal cancer, renal cancer, ovarian cancer, uterine cancer, prostate cancer, thyroid cancer, brain tumor, Kaposi's sarcoma, angioma, leukemia, polycythemia vera, neuroblastoma, retinoblastoma, myeloma, urinary bladder tumor, sarcoma, osteosarcoma, myosarcoma, skin cancer, basal cell carcinoma, skin appendage carcinoma, skin metastatic cancer, skin melanoma and so on. Preferred are colorectal cancer, gastric cancer, bladder cancer, renal cancer, uterine cancer and breast cancer, and more preferred are colorectal cancer, breast cancer and uterine cancer.

The pharmaceutical composition of the present invention may be administered in any mode, including oral administration, parenteral administration (e.g., subcutaneous administration, intracutaneous administration, mucosal administration, intrarectal administration, intravaginal administration, topical administration to the affected area, dermal administration), or direct administration to the affected area, etc.

The dose of the pharmaceutical composition of the present invention may generally be determined as appropriate for the age and body weight of a subject (patient) to be administered, the type and progression of disease, the route of administration, the frequency of administration, the period of administration, etc.

The dose of the pharmaceutical composition of the present invention may generally be determined as appropriate for the age and body weight of a subject (patient) to be administered, the type and progression of disease, the route of administration, the frequency of administration, the period of administration, etc., in consideration of the mixing ratio of the active ingredient in the formulation. Such an active ingredient may be exemplified by a chemotherapeutic agent, a toxin or a radioisotope, which is to be conjugated with the monoclonal antibody of the present invention, etc.

Detailed explanation will be given below for the case where the pharmaceutical composition of the present invention is used as a parenteral formulation.

For use as a parenteral formulation, the pharmaceutical composition of the present invention may usually be formulated into any dosage form, such as intravenous injections (including drip infusions), intramuscular injections, intraperitoneal injections, subcutaneous injections, suppositories, etc.

In the case of various types of injections, for example, they may be provided in the form of unit dose ampules or multi-dose containers or as freeze-dried powders which are dissolved again in a diluent before use. Such a parenteral formulation may comprise not only the active ingredient mentioned above (i.e., the anti-MCT5 antibody of the present invention or an antigen-binding fragment thereof), but also various known excipients and/or additives as appropriate for each dosage form as long as the effect of the above active ingredient is not impaired. In the case of various types of injections, examples of excipients and/or additives include water, glycerol, propylene glycol, and aliphatic polyalcohols such as polyethylene glycol, etc.

The dose (daily dose) of such a parenteral formulation is not limited in any way. For example in the case of various types of injections, the above active ingredient is generally used at a dose of preferably 1 to 15 mg/day, more preferably 2 to 12 mg/day, per kg body weight of a subject (patient) to be applied.

For use as an oral formulation, the pharmaceutical composition of the present invention may usually be formulated into any dosage form, such as tablets, capsules, granules, powders, pills, troches, solutions for internal use, suspensions, emulsions, syrups, etc., or may be formulated into a dried product which is dissolved again before use.

The pharmaceutical composition of the present invention may optionally comprise pharmaceutically acceptable additives. Specific examples of pharmaceutically acceptable additives include, but are not limited to, antioxidants, preservatives, colorants, flavors, diluents, emulsifiers, suspending agents, solvents, fillers, extenders, buffering agents, delivery vehicles, diluents, carriers, excipients and/or pharmaceutical adjuvants, etc.

The present invention provides a kit comprising the anti-MCT5 antibody of the present invention for use in the treatment of cancers, e.g., colorectal cancer. The kit of the present invention has no particular limitation on its constituent materials as long as it comprises the anti-MCT5 antibody of the present invention. In addition to the anti-MCT5 antibody of the present invention, the kit of the present invention may further comprise water, a buffer, a container, a syringe, an instruction manual and so on. The anti-MCT5 antibody of the present invention is provided, e.g., in an aqueous solution state or in a freeze-dried state, and may be reconstituted into an appropriate state before use. By using the kit of the present invention, cancers can be treated effectively.

Moreover, the present invention also provides a method for cancer treatment, which comprises administering the anti-MCT5 antibody of the present invention to a subject (e.g., a human subject). In addition, the present invention provides the anti-MCT5 antibody of the present invention for use in cancer treatment. As to the dose and mode of administration of the anti-MCT5 antibody, reference may be made to the descriptions about the pharmaceutical composition of the present invention.

The present invention also provides a method for cancer treatment, which comprises administering the monoclonal antibody of the present invention to a subject. In the method for cancer treatment of the present invention, the monoclonal antibody of the present invention may form a conjugate with a chemotherapeutic agent, a toxin or a radioisotope. Namely, the method for cancer treatment of the present invention may comprise administering the antibody conjugate of the present invention to a subject. Moreover, the method for cancer treatment of the present invention may be used in combination with an additional anticancer agent(s).

The dose of the pharmaceutical composition of the present invention may generally be determined as appropriate for the age and body weight of a subject (patient) to be administered, the type and progression of disease, the route of administration, the frequency of administration, the period of administration, etc.

Detailed explanation will be given below for the case where the pharmaceutical composition of the present invention is used as a parenteral formulation.

For use as a parenteral formulation, the pharmaceutical composition of the present invention may usually be formulated into any dosage form, such as intravenous injections (including drip infusions), intramuscular injections, intraperitoneal injections, subcutaneous injections, suppositories, etc.

In the case of various types of injections, for example, they may be provided in the form of unit dose ampules or multi-dose containers or as freeze-dried powders which are dissolved again in a diluent before use. Such a parenteral formulation may comprise not only the active ingredient mentioned above (i.e., the anti-MCT5 antibody of the present invention or an antigen-binding fragment thereof), but also various known excipients and/or additives as appropriate for each dosage form as long as the effect of the above active ingredient is not impaired. In the case of various types of injections, examples of excipients and/or additives include water, glycerol, propylene glycol, and aliphatic polyalcohols such as polyethylene glycol, etc.

The dose (daily dose) of such a parenteral formulation is not limited in any way. For example in the case of various types of injections, the above active ingredient is generally used at a dose of preferably 1 to 15 mg/day, more preferably 2 to 12 mg/day, per kg body weight of a subject (patient) to be applied.

For use as an oral formulation, the pharmaceutical composition of the present invention may usually be formulated into any dosage form, such as tablets, capsules, granules, powders, pills, troches, solutions for internal use, suspensions, emulsions, syrups, etc., or may be formulated into a dried product which is dissolved again before use.

### EXAMPLES

The present invention will be further described in more detail by way of the following examples, although the present invention is not limited only to these examples.

### Example 1

### (1) Cells

HCT116 cells were obtained from DS Pharma, while MDA-MB231 cells were obtained from American Type Culture collection (ATCC Accession No. HTB-26). As to MCF7 cells engineered to overexpress MCT5, the cells disclosed in Japanese Patent Application No. 2014-203162 were used for this purpose.

### (2) Monoclonal antibodies

### (i) Collection of antibody-producing cells

Clone No. IMI4C4a, which is a monoclonal antibody binding to MCT5, can be obtained when MCT5 or a partial peptide thereof is administered either alone or together with a carrier or diluent to immunize mammals. For example, a region consisting of amino acids at positions 196 to 299 (SEQ ID NO: 32) of MCT5 may be used for this purpose. This region is hereinafter referred to as "ICD4." The amount of the antigen to be administered per animal, the type of adjuvant to be used, the method of immunization and the interval between immunizations are the same as those used for preparation of polyclonal antibodies. After 1 to 30 days, preferably 2 to 5 days, from the final immunization date, animals showing antibody titers may be selected to collect antibody-producing cells. Antibody-producing cells may be exemplified by spleen cells, lymph node cells, peripheral blood cells and so on, with spleen cells or lymph node cells being preferred.
ICD4 amino acid sequence (SEQ ID NO: 32) 
RPIHIKSENNSGIKDKGSSLSAHGPEAHATETHCHETEESTIKDSTTQKAGLPSKN
LTVSQNQSEEFYNGPNRNRLLLKSDEESDKVISWSCKQLFDISLFRNP
ICD4 nucleotide sequence (SEQ ID NO: 31)

agacccatccatatcaaaagtgagaacaattctggtattaaagataaaggcagcagtttgtctgcacatggtccagaggcacat
gcaacagaaacacactgccatgagacagaagagtctaccatcaaggacagtactacgcagaaggctggactacctagcaaa

aatttaacagtctcacaaaatcaaagtgaagagttctacaatgggcctaacaggaacagactgttattaaagagtgatgaagaa
agtgataaggttatttcgtggagctgcaaacaactgtttgacatttctctctttagaaatcct

### (ii) Cell fusion

To obtain hybridomas, cell fusion is conducted between antibody-producing cells and myeloma cells. Operations for cell fusion may be accomplished in a known manner, for example, according to the method of Kohler et al. As myeloma cells to be fused with antibody-producing cells, it is possible to use generally available established cell lines of mouse or other animal origin. Cell lines preferred for use are those having drug selectivity and having the property of not surviving in HAT selective medium (i.e., a medium containing hypoxanthine, aminopterin and thymidine) in an unfused state, but surviving only when fused with antibody-producing cells. Examples of myeloma cells include mouse myeloma cell lines (e.g., P3X63-Ag8, P3X63-Ag8U.1, SP2/O-Ag14, PAI, P3U1, NSI/1-Ag4-1, NSO/1) and rat myeloma cell lines (e.g., YB2/0), etc.

Cell fusion between the above myeloma cells and antibody-producing cells may be accomplished as follows: in a serum-free medium for animal cell culture (e.g., DMEM or RPMI-1640 medium), 1 × 10⁸ to 5 × 10⁸ antibody-producing cells may be mixed with 2 × 10⁷ to 10 × 10⁷ myeloma cells (the ratio of antibody-producing cells to myeloma cells is 10:1 to 1:1) to cause fusion reaction in the presence of a cell fusion promoter. As a cell fusion promoter, it is possible to use polyethylene glycol having an average molecular weight of 1000 to 6000 daltons or Sendai virus, etc. Alternatively, a commercially available cell fusion apparatus using electrical stimulation (e.g., electroporation) may also be used to cause cell fusion between antibody-producing cells and myeloma cells.

### (iii) Screening and cloning of hybridomas

After cell fusion, the cells are screened to select desired hybridomas. For screening, a cell suspension may be diluted as appropriate with, e.g., RPMI-1640 medium containing 10% to 20% fetal bovine serum and then seeded by limiting dilution in microtiter plates at a density of about 0.3 cells/well by calculation, and a selective medium (e.g., HAT medium) may be added to each well, followed by culture while replacing the selective medium as appropriate. As a result, cells growing at around 10 days after the initiation of culture in the selective medium may be obtained as hybridomas.

Subsequently, the growing hybridomas are further screened. Screening of these hybridomas is not limited in any way and may be conducted in accordance with commonly used procedures. For example, aliquots of the culture supernatants contained in the wells where hybridomas have been cultured may be sampled and screened by enzyme immunoassay, radioimmunoassay, etc. More specifically, an antigen is adsorbed to 96-well plates, and the plates are then blocked with calf serum, skimmed milk, etc. The culture supernatants of hybridoma cells are reacted with the immobilized antigen at 37°C for 1 hour and then reacted with peroxidase labeled anti-mouse IgG at 37°C for 1 hour, followed by color development using orthophenylenediamine as a substrate. After the reaction is stopped with an acid, the plates are measured for absorbance at a wavelength of 490 nm for screening purposes.

Monoclonal antibody-producing hybridomas found to be positive when measured in the above manner are cloned by limiting dilution or other techniques to thereby finally establish hybridomas which are cells producing monoclonal antibodies specifically binding to MCT5.

### (iv) Collection of monoclonal antibodies

For collection of monoclonal antibodies from the establish hybridomas, commonly used procedures, e.g., cell culture-based procedures or ascites formation procedures may be used for this purpose. In cell culture-based procedures, hybridomas are cultured in an animal cell culture medium (e.g., 10% fetal bovine serum-containing RPMI-1640 medium, MEM medium or serum-free medium) under standard culture conditions (e.g., 37°C, 5% CO₂ concentration) for 7 to 14 days, and antibodies are obtained from their culture supernatants. In the case of ascites formation procedures, hybridomas are intraperitoneally administered at about 5 × 10⁶ to 2 × 10⁷ cells to animals of the same species as the mammal from which myeloma cells are derived, e.g., mice (BALB/c), whereby the hybridomas are allowed to grow in abundance. Then, their ascites are collected after 1 to 2 weeks. In cases where antibodies are required to be purified in the above antibody collection procedures, known techniques such as salting out with ammonium sulfate, ion exchange chromatography, gel filtration, affinity chromatography and so on may be selected as appropriate or used in combination for purification purposes.

### (3) ELISA

Blood samples which had been collected from mice through their tail veins and diluted 1/160,000 with PBS(-), or alternatively, culture supernatants collected from wells in which hybridomas were growing were analyzed for their antibody titer. ICD4 was diluted with PBS(-) and dispensed into 96-well ELISA plates (Nunc) in an amount of 50 ng per well, and immobilized onto the plate surface by being allowed to stand at room temperature for 1 hour. Then, after the plates were washed three times with 350 µL of TBS-T (25 mM Tris, 150 mM NaCl, 0.05% (v/v) Tween 20, pH 7.4), PBS-T containing 5% skimmed milk was dispensed in a volume of 300 µL per well to conduct blocking for 1 hour at room temperature. After washing with TBS-T, the diluted blood samples or the culture supernatants were dispensed into the ICD4-immobilized ELISA plates and reacted for 1 hour at room temperature. After washing with TBS-T, a 10,000-fold dilution of peroxidase (hereinafter abbreviated as POD)-labeled anti-mouse immunoglobulin antibody (BETHYL) was dispensed in a volume of 100 µL per well and reacted for 1 hour at room temperature. After washing in the same manner, OPD substrate prepared at 0.5 mg/mL POD was added to cause color development at room temperature for 5 minutes. After the reaction was stopped with 1.5 N sulfuric acid, the plates were measured for absorbance at 490 nm with a plate reader (Molecular Devices). Figure 1 shows the results obtained for the monoclonal antibodies prepared using ICD4 as an antigen by ELISA analysis on the culture supernatants of their respective hybridoma cells. These clones were singly cloned by limiting dilution and provided for use in the subsequent experiments.

### (4) Immunocytological staining

MCF7-MCT5, MDA-MB231 and HCT116 cells were cultured to reach 80% confluency and seeded at 1 × 10⁴ cells/well onto glass bottom dishes (MATSUNAMI) coated with Cellmatrix type I-A (Nitta Gelatin Inc., Japan). After culture for 1 day, the antibody IMI4C4a shown in (2) above was diluted to 10 µg/mL in fresh medium and the resulting solution was added to the cells and reacted on ice for 1 hour. After washing with fresh medium containing 10% FBS, anti-mouse IgG polyclonal antibody-Alexa Fluor 488 label or anti-mouse IgG polyclonal antibody-Alexa Fluor 555 label was reacted as a secondary antibody on ice for 30 minutes. After washing three times with fresh medium, the dishes were washed with PBS(-) and analyzed under a fluorescence microscope (BZ-8000, Keyence Corporation, Japan).

Figure 2 shows the results obtained when intact cells were stained with the antibody IMI4C4a for each case. Since the MCF7-MCT5 cells have been engineered to overexpress EGFP simultaneously with MCT5, intracellular EGFP fluorescence can be observed upon excitation at 488 nm. For this reason, anti-mouse IgG polyclonal antibody-Alexa Fluor 555 label was used as a secondary antibody for the MCF7-MCT5 cells. In all the cases, the antibody IMI4C4a was found to bind to the cell surface.

### (5) Flow cytometry analysis

HCT116 cells (which are human colorectal cancer cells) were cultured to reach 90% confluency. After being washed twice with PBS(-), the cells were collected with Accutase (Life Technologies) and suspended at 4 × 10⁵ cells in 100 mL of PBS(-) containing 1% FBS. To this suspension, the antibody IMI4C4a was added at a final concentration of 10 mg/mL and reacted on ice for 60 minutes. For use as a control, a cell suspension free from the antibody IMI4C4a was prepared. After the cells were washed twice with PBS + 1% PBS, a 1/1,1000 dilution of anti-mouse IgG polyclonal antibody-Alexa Fluor 488 label was added as a secondary antibody and reacted on ice for 30 minutes, and the cells were then washed twice with PBS containing 1% FBS, followed by analysis with a FACS Calibur (BD). The results obtained are shown in Figure 3.

As can be seen from Figure 3, the antibody IMI4C4a was found to strongly react with intact HCT116 cells, thus confirming that the anti-MCT5 antibody of the present invention recognizes MCT5 in its native structure.

### Example 2

### (1) Analysis of internalization

Analysis was conducted to determine whether the antibody IMI4C4a, which is an anti-MCT5 monoclonal antibody as shown in Example 1 above, was taken up into cells through internalization after binding onto the cell surface.

SK-BR3 and MDA-MB231 cells (which are human breast cancer cells) and HCT116 cells (which are human colorectal cancer cells) were seeded at 1 × 10⁴ cells/well onto glass bottom dishes. After culture for 1 day, the cells were cooled by being allowed to stand on ice for 15 minutes. After removal of the medium, the antibody IMI4C4a prepared at 10 mg/mL in fresh medium was added as a primary antibody and reacted on ice for 30 minutes. After being washed twice with fresh medium, the cells were reacted on ice for 30 minutes with anti-mouse IgG polyclonal antibody-Alexa Fluor 488 label as a secondary antibody. After being washed twice with fresh medium, the cells were cultured at 37°C under 5% CO₂ for 0, 15, 30, 60 or 120 minutes and then washed with fresh medium which had been cooled on ice. Further, to stain lysosomes in the cells, LysoTracker DND-99 (Life Technologies) prepared at 200 nM in PBS was added and reacted at room temperature for 1 minute. After being washed twice with PBS(-), the cells were analyzed under a fluorescence microscope. The results obtained are shown in Figure 4.

On the other hand, trastuzumab (preparation name: Herceptin), which is an anti-HER2 monoclonal antibody, was used to conduct the same analysis as shown above by using strongly HER2-positive SK-BR3 cells. The results obtained are also shown in Figure 3. In this case, for use as a secondary antibody, anti-human IgG polyclonal antibody-Alexa Fluor 488 label was used at the same concentration.

Figure 4 indicated that the antibody IMI4C4a was taken up into cells in all the cases using SK-BR3, MDA-MB231 and HCT116 cells.

Trastuzumab shown in the left panel in Figure 4 is an antibody which has been identified to be internalized (green fluorescence). In the case of trastuzumab, the antibody on the cell surface remains on the cell surface until 30 minutes and is then taken up into cells and rapidly taken up into lysosomes (red fluorescence), so that yellow fluorescence is observed. On the other hand, in the case of the antibody IMI4C4a, its uptake into cells was observed at 15 minutes after the initiation of culture and was found to proceed over time during 120 minutes, but its localization into lysosomes was not clearly detected.

### (2) Analysis of internalization mechanism

Internalization is mediated by cellular endocytosis. Endocytosis is classified into the following major types, i.e., clathrin-dependent endocytosis, caveolae-dependent endocytosis, phagocytosis and pinocytosis, depending on the type and size of substances to be taken up, differences in cellular molecules to be involved, etc.

Membrane proteins such as receptors are often taken up through clathrin-dependent endocytosis, and endocytosis inhibitors are used for their analysis.

Concanavalin A (ConA) serving as an inhibitor of clathrin-dependent endocytosis was used to analyze whether the internalization of the antibody IMI4C4a observed in Example (1) above was inhibited.

To MDA-MB231 cells cultured on glass bottom dishes in the same manner as shown in (1) above, a medium supplemented with ConA at 0.5 mg/mL was added and cultured at 37°C under 5% CO₂ for 60 minutes. After being washed twice with fresh medium, the cells were stained with a primary antibody and a secondary antibody in the same manner as shown above, and then analyzed under a fluorescence microscope after 0, 30, 60 and 120 minutes.

Figure 5 indicates that pre-treatment with ConA inhibits the internalization of the antibody IMI4C4a during the period observed. The left panel (0 mg/mL) shows time-dependent changes in the absence of ConA, while the right panels show the results obtained in the presence of ConA. The results indicated that pre-treatment with ConA allowed the antibody to remain on the cell surface even during the culture period where the antibody should have been transported into the cells. This confirmed that the antibody IMI4C4a bound to MCT5 on the cell membrane was taken up into the cells through clathrin-dependent endocytosis.

Moreover, further analysis was conducted to determine the reactivity with HCT116 cells which had been treated with ConA at a concentration of 0.5 mg/ml for 60 minutes. Antibodies IMI4C4a, 107, 217, 221, 223, 303, 306 and 301, each being prepared at 10 mg/ml, were reacted with the cells, followed by detection using anti-mouse IgG polyclonal antibody-Alexa Fluor 488 label as a secondary antibody. The experimental results obtained are shown in Figure 6. All the antibodies, except for the antibody 301, were found to bind to the surface of the HCT116 cells.

As shown in Example 1(3) above, the antibodies used are all antibodies binding to ICD4, thus suggesting that the antibody 301 would be an antibody binding to ICD4 but not to cells.

Trastuzumab, which has been extensively analyzed as an internalizing antibody, is reported to undergo turnover where the antibody bound to the extracellular surface is internalized but is rapidly returned to the extracellular surface. For this reason, it is deemed that internalization was not be able to be clearly detected during culture for 0 to 30 minutes, as shown in the right panel of Figure 3. On the other hand, in the case of IMI4C4a, internalization was able to be clearly detected even upon culture for 15 minutes; and hence its uptake efficiency is determined to be very good. Namely, cancer cells would be able to be killed with this antibody-drug conjugate even at low concentration.

### Example 3

### (1) Antibody biotinylation

The antibody IMI4C4a was biotinylated using EZ-link NHS-LC-LC-Biotin (PIERCE) in accordance with the instruction manual attached to the kit. The antibody IMI4C4a was prepared at 1 mg/mL in PBS(-), and NHS-LC-LC-Biotin powder was dissolved in DMSO and added to the antibody solution. After reaction for 30 minutes at room temperature, 1/100 volumes of 1 M Tris-HCl (pH 7.4) was added to deactivate the unreacted NHS-LC-LC-Biotin reagent. The unreacted reagent was removed off with Amicon Ultra 30 kDa (Millipore), and also the solvent was replaced with PBS(-).

### (2) Effect produced by the use of Streptavidin-ZAP

Analysis was conducted to determine whether the proliferation of cancer cells was able to be suppressed by means of the antibody's property of being taken up into cells. A medium was supplemented with 50 nM biotinylated antibody IMI4C4a, 303, 306 or 107 and then mixed with 15, 30 or 50 nM Streptavidin-ZAP (Advanced Targeting Systems, a Saporin-Streptavidin conjugate), followed by reaction on ice for 1 hour. The resulting solutions were each added to MCF7-MCT5 cells which had been seeded at 2,500 cells/well on the day before the analysis. Saporin has ribosome-inactivating activity and suppresses cell proliferation only when taken up into cells. After addition of each solution, the cells were cultured at 37°C under 5% CO₂ for 3 days and their proliferation was measured using WST-8 (Roche). The results obtained are shown in Figure 7.

As can be seen from Figure 7, when Streptavidin-ZAP was added in increasing amounts to a fixed amount of the biotinylated antibodies, the antibody IMI4C4a showed significant inhibition of cell proliferation. Moreover, as shown in Example 2(2), the antibody 301 is an antibody binding to its antigen ICD4 but not to cells. For this reason, the antibody 301 showed little suppression of cell proliferation even at increased concentrations of Streptavidin-ZAP in Figure 7.

The foregoing results indicate that the antibody IMI4C4a can be used as an antibody-drug conjugate for the purpose of killing cells.

### INDUSTRIAL APPLICABILITY

The present invention provides a monoclonal antibody specifically binding to an extracellular region of MCT5. The antibody of the present invention can be used for cancer treatment because of specifically binding to MCT5-expressing cancer cells and transporting a molecule conjugated to the antibody into the cells.

### Sequence Listing Free Text

SEQ ID NOs: 7 to 9, 12 to 14 and 21 to 30: synthetic sequences

## Claims

1. A monoclonal antibody against MCT5 or a fragment thereof.

2. The antibody according to claim 1, wherein the fragment of MCT5 is a fragment of an extracellular region of MCT5.

3. The antibody according to claim 1 or 2, wherein the fragment of the extracellular region of MCT5 consists of the amino acid sequence shown in SEQ ID NO: 4.

4. The antibody according to any one of claims 1 to 3, wherein the antibody is a chimeric antibody, a reshaped human antibody or a humanized antibody.

5. An antibody binding to an antigenic determinant to which the antibody according to any one of claims 1 to 4 binds.

6. A fragment of the antibody according to any one of claims 1 to 5.

7. A hybridoma producing the antibody according to any one of claims 1 to 4.

8. A pharmaceutical composition for use in cancer treatment, which comprises the antibody according to any one of claims 1 to 5 or the fragment according to claim 6.

9. A pharmaceutical composition for use in cancer treatment, which comprises a combination of the antibody according to any one of claims 1 to 5 or the fragment according to claim 6, and a chemotherapeutic agent, a toxin or a radioisotope.

10. A pharmaceutical composition for use in cancer treatment, which comprises a conjugate of the antibody according to any one of claims 1 to 5 or the fragment according to claim 6 with a chemotherapeutic agent, a toxin or a radioisotope.


SEQUENCE LISTING

<110>  Order-Made Medical Research Inc.

<120>  Pharmaceutical composition for treating cancer comprising an
       anti-MCT5 antibody

<130>  G1443WO

<150>  JP 2016-076565
<151>  2016-04-06

<160>  32

<170>  PatentIn version 3.5

<210>  1
<211>  1464
<212>  DNA
<213>  Homo sapiens



<220>
<221>  CDS
<222>  (1)..(1461)

<400>  1
atg ctg aag agg gag ggg aag gtc caa cct tac act aaa acc ctg gat        48
Met Leu Lys Arg Glu Gly Lys Val Gln Pro Tyr Thr Lys Thr Leu Asp
1               5                   10                  15

gga gga tgg gga tgg atg att gtg att cat ttt ttc ctg gtg aat gtg        96
Gly Gly Trp Gly Trp Met Ile Val Ile His Phe Phe Leu Val Asn Val
            20                  25                  30

ttt gtg atg ggg atg acc aag act ttt gca att ttc ttt gtg gtc ttt       144
Phe Val Met Gly Met Thr Lys Thr Phe Ala Ile Phe Phe Val Val Phe
        35                  40                  45

caa gaa gag ttt gaa ggc acc tca gag caa att ggt tgg att gga tcc       192
Gln Glu Glu Phe Glu Gly Thr Ser Glu Gln Ile Gly Trp Ile Gly Ser
    50                  55                  60

atc atg tca tct ctt cgt ttt tgt gca ggt ccc ctg gtt gct att att       240
Ile Met Ser Ser Leu Arg Phe Cys Ala Gly Pro Leu Val Ala Ile Ile
65                  70                  75                  80

tgt gac ata ctt gga gag aaa act acc tcc att ctt ggg gct ttc gtt       288
Cys Asp Ile Leu Gly Glu Lys Thr Thr Ser Ile Leu Gly Ala Phe Val
                85                  90                  95

gtt act ggt gga tat ctg atc agc agc tgg gcc aca agt att cct ttt       336
Val Thr Gly Gly Tyr Leu Ile Ser Ser Trp Ala Thr Ser Ile Pro Phe
            100                 105                 110

ctt tgt gtg act atg gga ctt cta ccc ggt ttg ggt tct gct ttc tta       384
Leu Cys Val Thr Met Gly Leu Leu Pro Gly Leu Gly Ser Ala Phe Leu
        115                 120                 125

tac caa gtg gct gct gtg gta act acc aaa tac ttc aaa aaa cga ttg       432
Tyr Gln Val Ala Ala Val Val Thr Thr Lys Tyr Phe Lys Lys Arg Leu
    130                 135                 140

gct ctt tct aca gct att gcc cgt tct ggg atg gga ctg act ttt ctt     480
Ala Leu Ser Thr Ala Ile Ala Arg Ser Gly Met Gly Leu Thr Phe Leu
145                 150                 155                 160

ttg gca ccc ttt aca aaa ttc ctg ata gat ctg tat gac tgg aca gga     528
Leu Ala Pro Phe Thr Lys Phe Leu Ile Asp Leu Tyr Asp Trp Thr Gly
                165                 170                 175

gcc ctt ata tta ttt gga gct atc gca ttg aat ttg gtg cct tct agt     576
Ala Leu Ile Leu Phe Gly Ala Ile Ala Leu Asn Leu Val Pro Ser Ser
            180                 185                 190

atg ctc tta aga ccc atc cat atc aaa agt gag aac aat tct ggt att     624
Met Leu Leu Arg Pro Ile His Ile Lys Ser Glu Asn Asn Ser Gly Ile
        195                 200                 205

aaa gat aaa ggc agc agt ttg tct gca cat ggt cca gag gca cat gca     672
Lys Asp Lys Gly Ser Ser Leu Ser Ala His Gly Pro Glu Ala His Ala
    210                 215                 220

aca gaa aca cac tgc cat gag aca gaa gag tct acc atc aag gac agt     720
Thr Glu Thr His Cys His Glu Thr Glu Glu Ser Thr Ile Lys Asp Ser
225                 230                 235                 240

act acg cag aag gct gga cta cct agc aaa aat tta aca gtc tca caa     768
Thr Thr Gln Lys Ala Gly Leu Pro Ser Lys Asn Leu Thr Val Ser Gln
                245                 250                 255

aat caa agt gaa gag ttc tac aat ggg cct aac agg aac aga ctg tta     816
Asn Gln Ser Glu Glu Phe Tyr Asn Gly Pro Asn Arg Asn Arg Leu Leu
            260                 265                 270

tta aag agt gat gaa gaa agt gat aag gtt att tcg tgg agc tgc aaa     864
Leu Lys Ser Asp Glu Glu Ser Asp Lys Val Ile Ser Trp Ser Cys Lys
        275                 280                 285

caa ctg ttt gac att tct ctc ttt aga aat cct ttc ttc tac ata ttt     912
Gln Leu Phe Asp Ile Ser Leu Phe Arg Asn Pro Phe Phe Tyr Ile Phe
    290                 295                 300

act tgg tct ttt ctc ctc agt cag tta gca tac ttc atc cct acc ttt     960
Thr Trp Ser Phe Leu Leu Ser Gln Leu Ala Tyr Phe Ile Pro Thr Phe
305                 310                 315                 320

cac ctg gta gcc aga gcc aaa aca ctg ggg att gac atc atg gat gcc    1008
His Leu Val Ala Arg Ala Lys Thr Leu Gly Ile Asp Ile Met Asp Ala
                325                 330                 335

tct tac ctt gtt tct gta gca ggt atc ctt gag acg gtc agt cag att    1056
Ser Tyr Leu Val Ser Val Ala Gly Ile Leu Glu Thr Val Ser Gln Ile
            340                 345                 350

att tct gga tgg gtt gct gat caa aac tgg att aag aag tat cat tac    1104
Ile Ser Gly Trp Val Ala Asp Gln Asn Trp Ile Lys Lys Tyr His Tyr
        355                 360                 365

cac aag tct tac ctc atc ctc tgc ggc atc act aac ctg ctt gct cct    1152
His Lys Ser Tyr Leu Ile Leu Cys Gly Ile Thr Asn Leu Leu Ala Pro
    370                 375                 380

tta gcc acc aca ttt cca cta ctt atg acc tac acc atc tgc ttt gcc    1200
Leu Ala Thr Thr Phe Pro Leu Leu Met Thr Tyr Thr Ile Cys Phe Ala
385                 390                 395                 400

atc ttt gct ggt ggt tac ctg gca ttg ata ctg cct gta ctg gtt gat 1248
Ile Phe Ala Gly Gly Tyr Leu Ala Leu Ile Leu Pro Val Leu Val Asp
                405                 410                 415

ctg tgt agg aat tct aca gta aac agg ttt ttg gga ctt gcc agt ttc 1296
Leu Cys Arg Asn Ser Thr Val Asn Arg Phe Leu Gly Leu Ala Ser Phe
            420                 425                 430

ttt gct ggg atg gct gtc ctt tct gga cca cct ata gca ggc tgg tta 1344
Phe Ala Gly Met Ala Val Leu Ser Gly Pro Pro Ile Ala Gly Trp Leu
        435                 440                 445

tat gat tat acc cag aca tac aat ggc tct ttc tac ttc tct ggc ata 1392
Tyr Asp Tyr Thr Gln Thr Tyr Asn Gly Ser Phe Tyr Phe Ser Gly Ile
    450                 455                 460

tgc tat ctc ctc tct tca gtt tcc ttt ttt ttt gta cca ttg gcc gaa 1440
Cys Tyr Leu Leu Ser Ser Val Ser Phe Phe Phe Val Pro Leu Ala Glu
465                 470                 475                 480

aga tgg aaa aac agt ctg acc tga 1464
Arg Trp Lys Asn Ser Leu Thr
                485

<210> 2
<211> 487
<212> PRT
<213> Homo sapiens

<400> 2

Met Leu Lys Arg Glu Gly Lys Val Gln Pro Tyr Thr Lys Thr Leu Asp
1               5                   10                  15

Gly Gly Trp Gly Trp Met Ile Val Ile His Phe Phe Leu Val Asn Val
            20                  25                  30

Phe Val Met Gly Met Thr Lys Thr Phe Ala Ile Phe Phe Val Val Phe
        35                  40                  45

Gln Glu Glu Phe Glu Gly Thr Ser Glu Gln Ile Gly Trp Ile Gly Ser
    50                  55                  60

Ile Met Ser Ser Leu Arg Phe Cys Ala Gly Pro Leu Val Ala Ile Ile
65                  70                  75                  80

Cys Asp Ile Leu Gly Glu Lys Thr Thr Ser Ile Leu Gly Ala Phe Val
                85                  90                  95

Val Thr Gly Gly Tyr Leu Ile Ser Ser Trp Ala Thr Ser Ile Pro Phe
            100                 105                 110

Leu Cys Val Thr Met Gly Leu Leu Pro Gly Leu Gly Ser Ala Phe Leu
        115                 120                 125

Tyr Gln Val Ala Ala Val Val Thr Thr Lys Tyr Phe Lys Lys Arg Leu
    130                 135                 140

Ala Leu Ser Thr Ala Ile Ala Arg Ser Gly Met Gly Leu Thr Phe Leu
145                 150                 155                 160

Leu Ala Pro Phe Thr Lys Phe Leu Ile Asp Leu Tyr Asp Trp Thr Gly
                165                 170                 175

Ala Leu Ile Leu Phe Gly Ala Ile Ala Leu Asn Leu Val Pro Ser Ser
            180                 185                 190

Met Leu Leu Arg Pro Ile His Ile Lys Ser Glu Asn Asn Ser Gly Ile
        195                 200                 205

Lys Asp Lys Gly Ser Ser Leu Ser Ala His Gly Pro Glu Ala His Ala
    210                 215                 220

Thr Glu Thr His Cys His Glu Thr Glu Glu Ser Thr Ile Lys Asp Ser
225                 230                 235                 240

Thr Thr Gln Lys Ala Gly Leu Pro Ser Lys Asn Leu Thr Val Ser Gln
                245                 250                 255

Asn Gln Ser Glu Glu Phe Tyr Asn Gly Pro Asn Arg Asn Arg Leu Leu
            260                 265                 270

Leu Lys Ser Asp Glu Glu Ser Asp Lys Val Ile Ser Trp Ser Cys Lys
        275                 280                 285

Gln Leu Phe Asp Ile Ser Leu Phe Arg Asn Pro Phe Phe Tyr Ile Phe
    290                 295                 300

Thr Trp Ser Phe Leu Leu Ser Gln Leu Ala Tyr Phe Ile Pro Thr Phe
305                 310                 315                 320

His Leu Val Ala Arg Ala Lys Thr Leu Gly Ile Asp Ile Met Asp Ala
                325                 330                 335

Ser Tyr Leu Val Ser Val Ala Gly Ile Leu Glu Thr Val Ser Gln Ile
            340                 345                 350

Ile Ser Gly Trp Val Ala Asp Gln Asn Trp Ile Lys Lys Tyr His Tyr
        355                 360                 365

His Lys Ser Tyr Leu Ile Leu Cys Gly Ile Thr Asn Leu Leu Ala Pro

370                 375                 380

Leu Ala Thr Thr Phe Pro Leu Leu Met Thr Tyr Thr Ile Cys Phe Ala
385                 390                 395                 400

Ile Phe Ala Gly Gly Tyr Leu Ala Leu Ile Leu Pro Val Leu Val Asp
                405                 410                 415

Leu Cys Arg Asn Ser Thr Val Asn Arg Phe Leu Gly Leu Ala Ser Phe
            420                 425                 430

Phe Ala Gly Met Ala Val Leu Ser Gly Pro Pro Ile Ala Gly Trp Leu
        435                 440                 445

Tyr Asp Tyr Thr Gln Thr Tyr Asn Gly Ser Phe Tyr Phe Ser Gly Ile
    450                 455                 460

Cys Tyr Leu Leu Ser Ser Val Ser Phe Phe Phe Val Pro Leu Ala Glu
465                 470                 475                 480

Arg Trp Lys Asn Ser Leu Thr
                485

<210>  3
<211>  24
<212>  DNA
<213>  Homo sapiens

<400>  3
aatttaacag tctcacaaaa tcaa                                              24

<210>  4
<211>  8
<212>  PRT
<213>  Homo sapiens

<400>  4

Asn Leu Thr Val Ser Gln Asn Gln
1               5

<210>  5
<211>  420
<212>  DNA
<213>  Homo sapiens

<220>
<221>  CDS
<222>  (1)..(420)

<400>  5
atg aaa tgc agc tgg gtc atc ttc ttc ctg atg gca gtg gtt aca ggg      48

Met Lys Cys Ser Trp Val Ile Phe Phe Leu Met Ala Val Val Thr Gly
1               5                   10                  15

gtc aat tca gag gtt cag ctg cag cag tct ggg gct gag ctt gtg agg      96
Val Asn Ser Glu Val Gln Leu Gln Gln Ser Gly Ala Glu Leu Val Arg
            20                  25                  30

cca ggg gcc tta gtc aag ttg tcc tgc aaa gct tct ggc ttc aac att      144
Pro Gly Ala Leu Val Lys Leu Ser Cys Lys Ala Ser Gly Phe Asn Ile
        35                  40                  45

aaa gac tac tat atg ttc tgg gtg aag cag agg cct gaa cag ggc ctg      192
Lys Asp Tyr Tyr Met Phe Trp Val Lys Gln Arg Pro Glu Gln Gly Leu
    50                  55                  60

gag tgg att gga tgg att gat cct gag aat ggt aat act ata ttt gac      240
Glu Trp Ile Gly Trp Ile Asp Pro Glu Asn Gly Asn Thr Ile Phe Asp
65                  70                  75                  80

ccg aag ttc cag ggc aag gcc agt ata aca gca gac aca tcc tcc aac      288
Pro Lys Phe Gln Gly Lys Ala Ser Ile Thr Ala Asp Thr Ser Ser Asn
                85                  90                  95

aca gcc tac ctg cag ctc agc agc ctg aca tct gag gac act gcc gtc      336
Thr Ala Tyr Leu Gln Leu Ser Ser Leu Thr Ser Glu Asp Thr Ala Val
            100                 105                 110

tat tac tgt gct aga atg att acg acc tat tac tat gct atg gac ttc      384
Tyr Tyr Cys Ala Arg Met Ile Thr Thr Tyr Tyr Tyr Ala Met Asp Phe
        115                 120                 125

tgg ggt caa gga acc tca gtc acc gtc tcc tca gcc                      420
Trp Gly Gln Gly Thr Ser Val Thr Val Ser Ser Ala
    130                 135                 140


<210>  6
<211>  140
<212>  PRT
<213>  Homo sapiens

<400>  6

Met Lys Cys Ser Trp Val Ile Phe Phe Leu Met Ala Val Val Thr Gly
1               5                   10                  15


Val Asn Ser Glu Val Gln Leu Gln Gln Ser Gly Ala Glu Leu Val Arg
            20                  25                  30


Pro Gly Ala Leu Val Lys Leu Ser Cys Lys Ala Ser Gly Phe Asn Ile
        35                  40                  45


Lys Asp Tyr Tyr Met Phe Trp Val Lys Gln Arg Pro Glu Gln Gly Leu
    50                  55                  60


Glu Trp Ile Gly Trp Ile Asp Pro Glu Asn Gly Asn Thr Ile Phe Asp
65                  70                  75                  80

Pro Lys Phe Gln Gly Lys Ala Ser Ile Thr Ala Asp Thr Ser Ser Asn
                85                  90                  95

Thr Ala Tyr Leu Gln Leu Ser Ser Leu Thr Ser Glu Asp Thr Ala Val
            100                 105                 110

Tyr Tyr Cys Ala Arg Met Ile Thr Thr Tyr Tyr Tyr Ala Met Asp Phe
        115                 120                 125

Trp Gly Gln Gly Thr Ser Val Thr Val Ser Ser Ala
    130                 135                 140

<210>  7
<211>  9
<212>  PRT
<213>  Artificial

<220>
<223>  Synthetic sequence

<400>  7

Phe Asn Ile Lys Asp Tyr Tyr Met Phe
1               5

<210>  8
<211>  18
<212>  PRT
<213>  Artificial

<220>
<223>  Synthetic sequence

<400>  8

Trp Ile Asp Pro Glu Asn Gly Asn Thr Ile Phe Asp Pro Lys Phe Gln
1               5                   10                  15

Gly Lys

<210>  9
<211>  12
<212>  PRT
<213>  Artificial

<220>
<223>  Synthetic sequence

<400>  9

Met Ile Thr Thr Tyr Tyr Tyr Ala Met Asp Phe Trp
1               5                   10

<210>  10

<211>   390
<212>   DNA
<213>   Homo sapiens

<220>
<221>   CDS
<222>   (1)..(390)

<400>   10
atg gcc tgg act cct ctc ttc ttc ttc ttt gtt ctt cat tgc tca ggt      48
Met Ala Trp Thr Pro Leu Phe Phe Phe Phe Val Leu His Cys Ser Gly
1               5                   10                  15

tct ttc tcc caa ctt gtg ctc act cag tca tct tca gcc tct ttc tcc      96
Ser Phe Ser Gln Leu Val Leu Thr Gln Ser Ser Ser Ala Ser Phe Ser
            20                  25                  30

ctg gga gcc tca gca aaa ctc acg tgc acc ttg agt agt cag cac agt      144
Leu Gly Ala Ser Ala Lys Leu Thr Cys Thr Leu Ser Ser Gln His Ser
        35                  40                  45

acg tac acc att gaa tgg tat cag caa cag cca ctc aag cct cct aag      192
Thr Tyr Thr Ile Glu Trp Tyr Gln Gln Gln Pro Leu Lys Pro Pro Lys
    50                  55                  60

tat gtg atg gag ctt aag aaa gat gga agc cac agc aca ggt gat ggg      240
Tyr Val Met Glu Leu Lys Lys Asp Gly Ser His Ser Thr Gly Asp Gly
65                  70                  75                  80

att cct gat cgc ttc tct gga tcc agc tct ggt gct gat cgc tac ctt      288
Ile Pro Asp Arg Phe Ser Gly Ser Ser Ser Gly Ala Asp Arg Tyr Leu
                85                  90                  95

agc gtt tcc agc atc cag cct gaa gat gaa gca ata tac atc tgt ggt      336
Ser Val Ser Ser Ile Gln Pro Glu Asp Glu Ala Ile Tyr Ile Cys Gly
            100                 105                 110

gtg ggt tat aca att aag gaa caa ttt gtg tat gtt ttc ggc ggt gga      384
Val Gly Tyr Thr Ile Lys Glu Gln Phe Val Tyr Val Phe Gly Gly Gly
        115                 120                 125

acc aag                                                              390
Thr Lys
    130


<210>   11
<211>   130
<212>   PRT
<213>   Homo sapiens

<400>   11

Met Ala Trp Thr Pro Leu Phe Phe Phe Phe Val Leu His Cys Ser Gly
1               5                   10                  15


Ser Phe Ser Gln Leu Val Leu Thr Gln Ser Ser Ser Ala Ser Phe Ser
            20                  25                  30


Leu Gly Ala Ser Ala Lys Leu Thr Cys Thr Leu Ser Ser Gln His Ser

35                  40                  45

Thr Tyr Thr Ile Glu Trp Tyr Gln Gln Gln Pro Leu Lys Pro Pro Lys
    50                  55                  60

Tyr Val Met Glu Leu Lys Lys Asp Gly Ser His Ser Thr Gly Asp Gly
65                  70                  75                  80

Ile Pro Asp Arg Phe Ser Gly Ser Ser Ser Gly Ala Asp Arg Tyr Leu
                85                  90                  95

Ser Val Ser Ser Ile Gln Pro Glu Asp Glu Ala Ile Tyr Ile Cys Gly
            100                 105                 110

Val Gly Tyr Thr Ile Lys Glu Gln Phe Val Tyr Val Phe Gly Gly Gly
        115                 120                 125

Thr Lys
    130

<210>  12
<211>  7
<212>  PRT
<213>  Artificial

<220>
<223>  Synthetic sequence

<400>  12

Ser Gln His Ser Thr Tyr Thr
1               5

<210>  13
<211>  10
<212>  PRT
<213>  Artificial

<220>
<223>  Synthetic sequence

<400>  13

Glu Leu Lys Lys Asp Gly Ser His Ser Thr
1               5                   10

<210>  14
<211>  7
<212>  PRT
<213>  Artificial

<220>
<223>  Synthetic sequence

<400>  14

Gly Tyr Thr Ile Lys Glu Gln
1               5


<210>  15
<211>  104
<212>  PRT
<213>  Homo sapiens

<400>  15

Arg Pro Ile His Ile Lys Ser Glu Asn Asn Ser Gly Ile Lys Asp Lys
1               5                   10                  15


Gly Ser Ser Leu Ser Ala His Gly Pro Glu Ala His Ala Thr Glu Thr
            20                  25                  30


His Cys His Glu Thr Glu Glu Ser Thr Ile Lys Asp Ser Thr Thr Gln
        35                  40                  45


Lys Ala Gly Leu Pro Ser Lys Asn Leu Thr Val Ser Gln Asn Gln Ser
    50                  55                  60


Glu Glu Phe Tyr Asn Gly Pro Asn Arg Asn Arg Leu Leu Leu Lys Ser
65                  70                  75                  80


Asp Glu Glu Ser Asp Lys Val Ile Ser Trp Ser Cys Lys Gln Leu Phe
                85                  90                  95


Asp Ile Ser Leu Phe Arg Asn Pro
            100


<210>  16
<211>  32
<212>  PRT
<213>  Homo sapiens

<400>  16

Thr His Cys His Glu Thr Glu Glu Ser Thr Ile Lys Asp Ser Thr Thr
1               5                   10                  15


Gln Lys Ala Gly Leu Pro Ser Lys Asn Leu Thr Val Ser Gln Asn Gln
            20                  25                  30


<210>  17
<211>  11
<212>  PRT
<213>  Homo sapiens

<400>  17

Gly Ser Ile Met Ser Ser Leu Arg Phe Cys Ala
1               5                   10

<210>  18
<211>  11
<212>  PRT
<213>  Homo sapiens

<400>  18

Met Gly Met Asp Asp Cys Asp Ser Phe Phe Pro
1               5                   10

<210>  19
<211>  10
<212>  PRT
<213>  Homo sapiens

<400>  19

Met Gly Met Asp Asp Cys Asp Ser Phe Phe
1               5                   10

<210>  20
<211>  47
<212>  PRT
<213>  Homo sapiens

<400>  20

Glu Ile Ile Pro Ser Phe Gln Ala Gly Tyr Met Ile Ile Pro Arg His
1               5                   10                  15

Thr Met Ala Leu Ser Thr Ser Leu Ala Tyr Ala Ile Ser Ser Leu Gln
            20                  25                  30

Phe Pro Phe Phe Leu Tyr His Trp Pro Lys Asp Gly Lys Thr Val
        35                  40                  45

<210>  21
<211>  27
<212>  DNA
<213>  Artificial

<220>
<223>  Synthetic sequence

<400>  21
ttcaacatta aagactacta tatgttc                                          27

<210>  22
<211>  54
<212>  DNA
<213>  Artificial

<220>

<223>  Synthetic sequence

<400>  22
tggattgatc ctgagaatgg taatactata tttgacccga agttccaggg caag          54


<210>  23
<211>  36
<212>  DNA
<213>  Artificial

<220>
<223>  Synthetic sequence

<400>  23
atgattacga cctattacta tgctatggac ttctgg                              36


<210>  24
<211>  21
<212>  DNA
<213>  Artificial

<220>
<223>  Synthetic sequence

<400>  24
agtcagcaca gtacgtacac c                                              21


<210>  25
<211>  30
<212>  DNA
<213>  Artificial

<220>
<223>  Synthetic sequence

<400>  25
gagcttaaga aagatggaag ccacagcaca                                     30


<210>  26
<211>  21
<212>  DNA
<213>  Artificial

<220>
<223>  Synthetic sequence

<400>  26
ggttatacaa ttaaggaaca a                                              21


<210>  27
<211>  1410
<212>  DNA
<213>  Artificial

<220>
<223>  Synthetic sequence

<220>
<221>  CDS
<222>  (1)..(1407)

<400>  27
atg aaa tgc agc tgg gtc atc ttc ttc ctg atg gca gtg gtt aca ggg      48
Met Lys Cys Ser Trp Val Ile Phe Phe Leu Met Ala Val Val Thr Gly
1               5                   10                  15

gtc aat tca gag gtt cag ctg cag cag tct ggg gct gag ctt gtg agg      96
Val Asn Ser Glu Val Gln Leu Gln Gln Ser Gly Ala Glu Leu Val Arg
            20                  25                  30

cca ggg gcc tta gtc aag ttg tcc tgc aaa gct tct ggc ttc aac att      144
Pro Gly Ala Leu Val Lys Leu Ser Cys Lys Ala Ser Gly Phe Asn Ile
        35                  40                  45

aaa gac tac tat atg ttc tgg gtg aag cag agg cct gaa cag ggc ctg      192
Lys Asp Tyr Tyr Met Phe Trp Val Lys Gln Arg Pro Glu Gln Gly Leu
    50                  55                  60

gag tgg att gga tgg att gat cct gag aat ggt aat act ata ttt gac      240
Glu Trp Ile Gly Trp Ile Asp Pro Glu Asn Gly Asn Thr Ile Phe Asp
65                  70                  75                  80

ccg aag ttc cag ggc aag gcc agt ata aca gca gac aca tcc tcc aac      288
Pro Lys Phe Gln Gly Lys Ala Ser Ile Thr Ala Asp Thr Ser Ser Asn
                85                  90                  95

aca gcc tac ctg cag ctc agc agc ctg aca tct gag gac act gcc gtc      336
Thr Ala Tyr Leu Gln Leu Ser Ser Leu Thr Ser Glu Asp Thr Ala Val
            100                 105                 110

tat tac tgt gct aga atg att acg acc tat tac tat gct atg gac ttc      384
Tyr Tyr Cys Ala Arg Met Ile Thr Thr Tyr Tyr Tyr Ala Met Asp Phe
        115                 120                 125

tgg ggt caa gga acc tca gtc acc gtc tcc gca gct agc acc aag ggc      432
Trp Gly Gln Gly Thr Ser Val Thr Val Ser Ala Ala Ser Thr Lys Gly
    130                 135                 140

cca tcg gtc ttc ccc ctg gca ccc tcc tcc aag agc acc tct ggg ggc      480
Pro Ser Val Phe Pro Leu Ala Pro Ser Ser Lys Ser Thr Ser Gly Gly
145                 150                 155                 160

aca gcg gcc ctg ggc tgc ctg gtc aag gac tac ttc ccc gaa ccg gtg      528
Thr Ala Ala Leu Gly Cys Leu Val Lys Asp Tyr Phe Pro Glu Pro Val
                165                 170                 175

acg gtg tcg tgg aac tca ggc gcc ctg acc agc ggc gtg cac acc ttc      576
Thr Val Ser Trp Asn Ser Gly Ala Leu Thr Ser Gly Val His Thr Phe
            180                 185                 190

ccg gct gtc cta cag tcc tca gga ctc tac tcc ctc agc agc gtg gtg      624
Pro Ala Val Leu Gln Ser Ser Gly Leu Tyr Ser Leu Ser Ser Val Val
        195                 200                 205

acc gtg ccc tcc agc agc ttg ggc acc cag acc tac atc tgc aac gtg      672
Thr Val Pro Ser Ser Ser Leu Gly Thr Gln Thr Tyr Ile Cys Asn Val
    210                 215                 220

aat cac aag ccc agc aac acc aag gtg gac aag aaa gtt gag ccc aaa      720
Asn His Lys Pro Ser Asn Thr Lys Val Asp Lys Lys Val Glu Pro Lys

225                 230                 235                 240

tct tgt gac aaa act cac aca tgc cca ccg tgc cca gca cct gaa ctc       768
Ser Cys Asp Lys Thr His Thr Cys Pro Pro Cys Pro Ala Pro Glu Leu
                245                 250                 255

ctg ggg gga ccg tca gtc ttc ctc ttc ccc cca aaa ccc aag gac acc       816
Leu Gly Gly Pro Ser Val Phe Leu Phe Pro Pro Lys Pro Lys Asp Thr
            260                 265                 270

ctc atg atc tcc cgg acc cct gag gtc aca tgc gtg gtg gtg gac gtg       864
Leu Met Ile Ser Arg Thr Pro Glu Val Thr Cys Val Val Val Asp Val
        275                 280                 285

agc cac gaa gac cct gag gtc aag ttc aac tgg tac gtg gac ggc gtg       912
Ser His Glu Asp Pro Glu Val Lys Phe Asn Trp Tyr Val Asp Gly Val
    290                 295                 300

gag gtg cat aat gcc aag aca aag ccg cgg gag gag cag tac aac agc       960
Glu Val His Asn Ala Lys Thr Lys Pro Arg Glu Glu Gln Tyr Asn Ser
305                 310                 315                 320

acg tac cgt gtg gtc agc gtc ctc acc gtc ctg cac cag gac tgg ctg      1008
Thr Tyr Arg Val Val Ser Val Leu Thr Val Leu His Gln Asp Trp Leu
                325                 330                 335

aat ggc aag gag tac aag tgc aag gtc tcc aac aaa gcc ctc cca gcc      1056
Asn Gly Lys Glu Tyr Lys Cys Lys Val Ser Asn Lys Ala Leu Pro Ala
            340                 345                 350

ccc atc gag aaa acc atc tcc aaa gcc aaa ggg cag ccc cga gaa cca      1104
Pro Ile Glu Lys Thr Ile Ser Lys Ala Lys Gly Gln Pro Arg Glu Pro
        355                 360                 365

cag gtg tac acc ctg ccc cca tcc cgg gat gag ctg acc aag aac cag      1152
Gln Val Tyr Thr Leu Pro Pro Ser Arg Asp Glu Leu Thr Lys Asn Gln
    370                 375                 380

gtc agc ctg acc tgc ctg gtc aaa ggc ttc tat ccc agc gac atc gcc      1200
Val Ser Leu Thr Cys Leu Val Lys Gly Phe Tyr Pro Ser Asp Ile Ala
385                 390                 395                 400

gtg gag tgg gag agc aat ggg cag ccg gag aac aac tac aag acc acg      1248
Val Glu Trp Glu Ser Asn Gly Gln Pro Glu Asn Asn Tyr Lys Thr Thr
                405                 410                 415

cct ccc gtg ctg gac tcc gac ggc tcc ttc ttc ctc tac agc aag ctc      1296
Pro Pro Val Leu Asp Ser Asp Gly Ser Phe Phe Leu Tyr Ser Lys Leu
            420                 425                 430

acc gtg gac aag agc agg tgg cag cag ggg aac gtc ttc tca tgc tcc      1344
Thr Val Asp Lys Ser Arg Trp Gln Gln Gly Asn Val Phe Ser Cys Ser
        435                 440                 445

gtg atg cat gag gct ctg cac aac cac tac acg cag aag agc ctc tcc      1392
Val Met His Glu Ala Leu His Asn His Tyr Thr Gln Lys Ser Leu Ser
    450                 455                 460

ctg tct ccg ggt aaa tga                                              1410
Leu Ser Pro Gly Lys
465

<210>  28
<211>  469
<212>  PRT
<213>  Artificial

<220>
<223>  Synthetic Construct

<400>  28

Met Lys Cys Ser Trp Val Ile Phe Phe Leu Met Ala Val Val Thr Gly
1               5                   10                  15

Val Asn Ser Glu Val Gln Leu Gln Gln Ser Gly Ala Glu Leu Val Arg
            20                  25                  30

Pro Gly Ala Leu Val Lys Leu Ser Cys Lys Ala Ser Gly Phe Asn Ile
        35                  40                  45

Lys Asp Tyr Tyr Met Phe Trp Val Lys Gln Arg Pro Glu Gln Gly Leu
    50                  55                  60

Glu Trp Ile Gly Trp Ile Asp Pro Glu Asn Gly Asn Thr Ile Phe Asp
65                  70                  75                  80

Pro Lys Phe Gln Gly Lys Ala Ser Ile Thr Ala Asp Thr Ser Ser Asn
                85                  90                  95

Thr Ala Tyr Leu Gln Leu Ser Ser Leu Thr Ser Glu Asp Thr Ala Val
            100                 105                 110

Tyr Tyr Cys Ala Arg Met Ile Thr Thr Tyr Tyr Tyr Ala Met Asp Phe
        115                 120                 125

Trp Gly Gln Gly Thr Ser Val Thr Val Ser Ala Ala Ser Thr Lys Gly
    130                 135                 140

Pro Ser Val Phe Pro Leu Ala Pro Ser Ser Lys Ser Thr Ser Gly Gly
145                 150                 155                 160

Thr Ala Ala Leu Gly Cys Leu Val Lys Asp Tyr Phe Pro Glu Pro Val
                165                 170                 175

Thr Val Ser Trp Asn Ser Gly Ala Leu Thr Ser Gly Val His Thr Phe
            180                 185                 190

Pro Ala Val Leu Gln Ser Ser Gly Leu Tyr Ser Leu Ser Ser Val Val
        195                 200                 205

Thr Val Pro Ser Ser Ser Leu Gly Thr Gln Thr Tyr Ile Cys Asn Val

210                 215                 220

Asn His Lys Pro Ser Asn Thr Lys Val Asp Lys Lys Val Glu Pro Lys
225                 230                 235                 240

Ser Cys Asp Lys Thr His Thr Cys Pro Pro Cys Pro Ala Pro Glu Leu
                245                 250                 255

Leu Gly Gly Pro Ser Val Phe Leu Phe Pro Pro Lys Pro Lys Asp Thr
            260                 265                 270

Leu Met Ile Ser Arg Thr Pro Glu Val Thr Cys Val Val Val Asp Val
        275                 280                 285

Ser His Glu Asp Pro Glu Val Lys Phe Asn Trp Tyr Val Asp Gly Val
    290                 295                 300

Glu Val His Asn Ala Lys Thr Lys Pro Arg Glu Glu Gln Tyr Asn Ser
305                 310                 315                 320

Thr Tyr Arg Val Val Ser Val Leu Thr Val Leu His Gln Asp Trp Leu
                325                 330                 335

Asn Gly Lys Glu Tyr Lys Cys Lys Val Ser Asn Lys Ala Leu Pro Ala
            340                 345                 350

Pro Ile Glu Lys Thr Ile Ser Lys Ala Lys Gly Gln Pro Arg Glu Pro
        355                 360                 365

Gln Val Tyr Thr Leu Pro Pro Ser Arg Asp Glu Leu Thr Lys Asn Gln
    370                 375                 380

Val Ser Leu Thr Cys Leu Val Lys Gly Phe Tyr Pro Ser Asp Ile Ala
385                 390                 395                 400

Val Glu Trp Glu Ser Asn Gly Gln Pro Glu Asn Asn Tyr Lys Thr Thr
                405                 410                 415

Pro Pro Val Leu Asp Ser Asp Gly Ser Phe Phe Leu Tyr Ser Lys Leu
            420                 425                 430

Thr Val Asp Lys Ser Arg Trp Gln Gln Gly Asn Val Phe Ser Cys Ser
        435                 440                 445

Val Met His Glu Ala Leu His Asn His Tyr Thr Gln Lys Ser Leu Ser
    450                 455                 460

Leu Ser Pro Gly Lys
465

<210> 29
<211> 723
<212> DNA
<213> Artificial

<220>
<223> Synthetic sequence

<220>
<221> CDS
<222> (1)..(720)

<400> 29
atg gcc tgg act cct ctc ttc ttc ttc ttt gtt ctt cat tgc tca ggt 48
Met Ala Trp Thr Pro Leu Phe Phe Phe Phe Val Leu His Cys Ser Gly
1               5                   10                  15

tct ttc tcc caa ctt gtg ctc act cag tca tct tca gcc tct ttc tcc 96
Ser Phe Ser Gln Leu Val Leu Thr Gln Ser Ser Ser Ala Ser Phe Ser
            20                  25                  30

ctg gga gcc tca gca aaa ctc acg tgc acc ttg agt agt cag cac agt 144
Leu Gly Ala Ser Ala Lys Leu Thr Cys Thr Leu Ser Ser Gln His Ser
        35                  40                  45

acg tac acc att gaa tgg tat cag caa cag cca ctc aag cct cct aag 192
Thr Tyr Thr Ile Glu Trp Tyr Gln Gln Gln Pro Leu Lys Pro Pro Lys
    50                  55                  60

tat gtg atg gag ctt aag aaa gat gga agc cac agc aca ggt gat ggg 240
Tyr Val Met Glu Leu Lys Lys Asp Gly Ser His Ser Thr Gly Asp Gly
65                  70                  75                  80

att cct gat cgc ttc tct gga tcc agc tct ggt gct gat cgc tac ctt 288
Ile Pro Asp Arg Phe Ser Gly Ser Ser Ser Gly Ala Asp Arg Tyr Leu
                85                  90                  95

agc gtt tcc agc atc cag cct gaa gat gaa gca ata tac atc tgt ggt 336
Ser Val Ser Ser Ile Gln Pro Glu Asp Glu Ala Ile Tyr Ile Cys Gly
            100                 105                 110

gtg ggt tat aca att aag gaa caa ttt gtg tat gtt ttc ggc ggt gga 384
Val Gly Tyr Thr Ile Lys Glu Gln Phe Val Tyr Val Phe Gly Gly Gly
        115                 120                 125

acc aag gtc act gtc cta ggt cag ccc aag gct gcc ccc tcg gtc act 432
Thr Lys Val Thr Val Leu Gly Gln Pro Lys Ala Ala Pro Ser Val Thr
    130                 135                 140

ctg ttc ccg ccc tcc tct gag gag ctt caa gcc aac aag gcc aca ctg 480
Leu Phe Pro Pro Ser Ser Glu Glu Leu Gln Ala Asn Lys Ala Thr Leu
145                 150                 155                 160

gtg tgt ctc ata agt gac ttc tac ccg gga gcc gtg aca gtg gcc tgg 528
Val Cys Leu Ile Ser Asp Phe Tyr Pro Gly Ala Val Thr Val Ala Trp
                165                 170                 175

aag gca gat agc agc ccc gtc aag gcg gga gtg gag acc acc aca ccc 576

Lys Ala Asp Ser Ser Pro Val Lys Ala Gly Val Glu Thr Thr Thr Pro
            180                 185                 190

tcc aaa caa agc aac aac aag tac gcg gcc agc agc tat ctg agc ctg       624
Ser Lys Gln Ser Asn Asn Lys Tyr Ala Ala Ser Ser Tyr Leu Ser Leu
        195                 200                 205

acg cct gag cag tgg aag tcc cac aga agc tac agc tgc cag gtc acg       672
Thr Pro Glu Gln Trp Lys Ser His Arg Ser Tyr Ser Cys Gln Val Thr
    210                 215                 220

cat gaa ggg agc acc gtg gag aag aca gtg gcc cct aca gaa tgt tca       720
His Glu Gly Ser Thr Val Glu Lys Thr Val Ala Pro Thr Glu Cys Ser
225                 230                 235                 240

tag                                                                   723


<210>  30
<211>  240
<212>  PRT
<213>  Artificial

<220>
<223>  Synthetic Construct

<400>  30

Met Ala Trp Thr Pro Leu Phe Phe Phe Phe Val Leu His Cys Ser Gly
1               5                   10                  15


Ser Phe Ser Gln Leu Val Leu Thr Gln Ser Ser Ser Ala Ser Phe Ser
            20                  25                  30


Leu Gly Ala Ser Ala Lys Leu Thr Cys Thr Leu Ser Ser Gln His Ser
        35                  40                  45


Thr Tyr Thr Ile Glu Trp Tyr Gln Gln Gln Pro Leu Lys Pro Pro Lys
    50                  55                  60


Tyr Val Met Glu Leu Lys Lys Asp Gly Ser His Ser Thr Gly Asp Gly
65                  70                  75                  80


Ile Pro Asp Arg Phe Ser Gly Ser Ser Ser Gly Ala Asp Arg Tyr Leu
                85                  90                  95


Ser Val Ser Ser Ile Gln Pro Glu Asp Glu Ala Ile Tyr Ile Cys Gly
            100                 105                 110


Val Gly Tyr Thr Ile Lys Glu Gln Phe Val Tyr Val Phe Gly Gly Gly
        115                 120                 125


Thr Lys Val Thr Val Leu Gly Gln Pro Lys Ala Ala Pro Ser Val Thr
    130                 135                 140

Leu Phe Pro Pro Ser Ser Glu Glu Leu Gln Ala Asn Lys Ala Thr Leu
145                 150                 155                 160

Val Cys Leu Ile Ser Asp Phe Tyr Pro Gly Ala Val Thr Val Ala Trp
                165                 170                 175

Lys Ala Asp Ser Ser Pro Val Lys Ala Gly Val Glu Thr Thr Thr Pro
            180                 185                 190

Ser Lys Gln Ser Asn Asn Lys Tyr Ala Ala Ser Ser Tyr Leu Ser Leu
        195                 200                 205

Thr Pro Glu Gln Trp Lys Ser His Arg Ser Tyr Ser Cys Gln Val Thr
    210                 215                 220

His Glu Gly Ser Thr Val Glu Lys Thr Val Ala Pro Thr Glu Cys Ser
225                 230                 235                 240

<210>  31
<211>  312
<212>  DNA
<213>  Homo sapiens

<220>
<221>  CDS
<222>  (1)..(312)

<400>  31
aga ccc atc cat atc aaa agt gag aac aat tct ggt att aaa gat aaa      48
Arg Pro Ile His Ile Lys Ser Glu Asn Asn Ser Gly Ile Lys Asp Lys
1               5                   10                  15

ggc agc agt ttg tct gca cat ggt cca gag gca cat gca aca gaa aca      96
Gly Ser Ser Leu Ser Ala His Gly Pro Glu Ala His Ala Thr Glu Thr
            20                  25                  30

cac tgc cat gag aca gaa gag tct acc atc aag gac agt act acg cag      144
His Cys His Glu Thr Glu Glu Ser Thr Ile Lys Asp Ser Thr Thr Gln
        35                  40                  45

aag gct gga cta cct agc aaa aat tta aca gtc tca caa aat caa agt      192
Lys Ala Gly Leu Pro Ser Lys Asn Leu Thr Val Ser Gln Asn Gln Ser
    50                  55                  60

gaa gag ttc tac aat ggg cct aac agg aac aga ctg tta tta aag agt      240
Glu Glu Phe Tyr Asn Gly Pro Asn Arg Asn Arg Leu Leu Leu Lys Ser
65                  70                  75                  80

gat gaa gaa agt gat aag gtt att tcg tgg agc tgc aaa caa ctg ttt      288
Asp Glu Glu Ser Asp Lys Val Ile Ser Trp Ser Cys Lys Gln Leu Phe
                85                  90                  95

gac att tct ctc ttt aga aat cct                                      312
Asp Ile Ser Leu Phe Arg Asn Pro
            100

<210>  32
<211>  104
<212>  PRT
<213>  Homo sapiens

<400>  32

Arg Pro Ile His Ile Lys Ser Glu Asn Asn Ser Gly Ile Lys Asp Lys
1               5                   10                  15

Gly Ser Ser Leu Ser Ala His Gly Pro Glu Ala His Ala Thr Glu Thr
            20                  25                  30

His Cys His Glu Thr Glu Glu Ser Thr Ile Lys Asp Ser Thr Thr Gln
        35                  40                  45

Lys Ala Gly Leu Pro Ser Lys Asn Leu Thr Val Ser Gln Asn Gln Ser
    50                  55                  60

Glu Glu Phe Tyr Asn Gly Pro Asn Arg Asn Arg Leu Leu Leu Lys Ser
65                  70                  75                  80

Asp Glu Glu Ser Asp Lys Val Ile Ser Trp Ser Cys Lys Gln Leu Phe
                85                  90                  95

Asp Ile Ser Leu Phe Arg Asn Pro
            100